# EUROPEAN PATENT APPLICATION

(11) **EP 4 706 711 A2**
(43) Date of publication of application: **11.03.2026**
(21) Application number: 25215348.1
(22) Date of filing: 22.06.2022
(51) Int. Cl.: A61M 15/00

(54) **DRY POWDER MEDICAMENT INHALER**

(30) Priority: 13.08.2021 GB 202111658
(62) Divisional of application: 24153100.3
(71) Applicant: Norton (Waterford) Limited, Waterford, X91 WK68 (IE)
(72) Inventor: CROWLEY, Peter John, Waterford (IE); HAZENBERG, Jan Geert, Co. Kilkenny (IE); BUCK, Daniel, Waterford (IE); GOTTESMAN, Josh, Bristol (GB)
(74) Representative: Elkington and Fife LLP

(57) **Abstract**

Provided is a dry powder inhaler for delivering medicament from at least one medicament carrier having a plurality of spaced-apart medicament doses. The inhaler comprises: a housing having regions for accommodating unused and used portions of the medicament carrier; a mouthpiece component extending from the housing and through which a user is able to inhale; a manifold arranged in the housing and defining at least part of an air flow path extending from an air inlet to the mouthpiece, the manifold further defining a dispensing position for the medicament carrier; a dispensing mechanism arranged in the housing for moving a medicament dose of the medicament carrier to the dispensing position and for placing the medicament dose in fluid communication with the air flow path ready for inhalation by the user; and a mouthpiece cover being rotatably connected to the housing for sequential movement about a rotation axis from a first position to a second position, and from the second position to a third position in which the mouthpiece component is completely uncovered.

The invention provides arrangements in which the coupling of the mouthpiece cover to the dispensing mechanism is arranged to be non-linear, such that movement of the mouthpiece cover from the first position to the second position causes the medicament dose to be moved with a first movement ratio, and movement of the mouthpiece cover from the second position to the third position causes the medicament dose to be moved with a second movement ratio which is different to the first movement ratio.

## Description

### FIELD OF THE INVENTION

This invention relates to a dry powder inhaler. In particular, this invention relates to a dry powder inhaler for delivering medicament from at least one medicament carrier having a plurality of spaced-apart medicament doses.

### BACKGROUND OF THE INVENTION

Inhalers for drug delivery to a patient by inhalation are well-known. Such devices include metered-dose inhalers and dry powder inhalers.

Metered dose inhalers typically comprise a container containing a propellant and a liquid solution or suspension of a medicament. Metered dose inhalers further include a dispensing valve which, when actuated, causes the medicament to be forced out of the container by expansion of the propellant in the form of an aerosol.

Dry powder inhalers, on the other hand, typically comprise a supply of the medicament in dry powder form, and are arranged to permit the user to inhale discrete doses from the supply of powder medicament.

Some dry powder inhalers comprise a bulk reservoir of powder medicament, with a dispensing mechanism being configured to separate a dose of the medicament from the reservoir and make it available for inhalation by the user. Other types of dry powder inhaler comprise a plurality of pre-metered doses of powder medicament in containers, for example in capsules or blisters, and a dispensing mechanism which is configured to open the containers and make the doses of medicament available for inhalation by the user.

One such type of dry powder inhaler comprises a medicament carrier having a plurality of spaced-apart medicament doses, for example in the form of an elongate blister strip having spaced-apart blisters. The inhaler comprises a manually-operated dispensing mechanism for moving a medicament dose of the medicament carrier to a dispensing position in the inhaler, and for placing the medicament dose in fluid communication with an air flow path of the inhaler, for example by piercing or peeling open the blisters, ready for inhalation by the user.

An inhaler of this type is described in GB 2242134 A. The dispensing mechanism of this device is operated by a lever, which causes a blister to be moved to the dispensing position of the inhaler and peeled open. Another inhaler of this type is described in WO 2007/012871 A1. The dispensing mechanism of this device is operated by a rotatable mouthpiece cover, the opening of which causes a blister to be moved to the dispensing position of the inhaler and peeled open.

**It** is noted that inhalers of this type can be used for combination therapy, whereby a plurality of different powder medicaments can be dispensed for simultaneous inhalation by the user. The different medicaments can be provided in groups of blisters of the medicament carrier, which can then be opened together. Alternatively, the different medicaments can be provided in different blister strips, with the dispensing mechanism simultaneously acting on all of the blister strips to open a blister of each strip together. Such use is advantageous where the different medicaments cannot be stored together, for example because of chemical incompatibilities.

A problem has been recognised in relation to inhalers of the type described above in that, when the dispensing mechanism is operated by opening a rotatable mouthpiece cover, there is a risk that the mouthpiece cover may unintentionally move prior to intended use of the inhaler, for example when being carried in a garment pocket. Such movement is undesirable because it can cause unintended partial actuation of the dispensing mechanism.

WO 2007/012871 A1 discloses an inhaler which is configured so that movement from a first position, in which the mouthpiece is covered, to a second position, in which the mouthpiece is at least partly uncovered, does not result in actuation of the dispensing mechanism. The dispensing mechanism is actuated by moving the mouthpiece cover from the second position to a third position, in which the mouthpiece is uncovered. WO 2007/012871 A1 teaches that such an arrangement allows for the mouthpiece to be cleaned without having to actuate the dispensing mechanism.

The inhaler taught by WO 2007/012871 A1 allows the mouthpiece cover to be unintentionally or accidentally moved between the first and second positions. This arrangement gives rise to a further problem, which is that the mouthpiece is likely to become partially uncovered during periods when the inhaler is not in use, which leaves the mouthpiece more susceptible to contamination. Such contamination can be unhygienic and can potentially affect the medicament delivery performance of the inhaler. Moreover, the user may not even be aware of such contamination, if the mouthpiece cover has moved accidentally or unintentionally.

Another problem is that the force required to open the mouthpiece cover of WO 2007/012871 A1, varies considerably as the mouthpiece cover is moved, with movement from the first position to the second position requiring relatively little force owing to the lack of any engagement with the dispensing mechanism, and then a great deal of force being required for movement from the second position to the third position, which drives the dispensing mechanism. This sudden increase in force when moving through the second position makes the inhaler more difficult to use, and the corresponding variation in resistance is typically perceived by the user as low quality. It also renders the mouthpiece cover and components of the dispensing mechanism more susceptible to breakage, and can potentially result in vibration-induced movement of the powder medicament, leading to poor dosing consistency and contamination of the internal components of the inhaler.

### SUMMARY OF THE INVENTION

According to a first aspect of the invention, there is provided a dry powder inhaler for delivering medicament from at least one medicament carrier having a plurality of spaced-apart medicament doses, the dry powder inhaler comprising:
a housing having regions for accommodating unused and used portions of the medicament carrier;
a mouthpiece component joined to the housing and through which a user is able to inhale, the mouthpiece component including surfaces for contacting the user's lips;
a dispensing mechanism arranged in the housing for moving a next medicament dose of the medicament carrier to a dispensing position; and
a mouthpiece cover rotatably connected to the housing for sequential movement about a rotation axis from a first position in which the mouthpiece component is completely covered to a second position, and from the second position to a third position in which the mouthpiece component is completely uncovered, the second position being between the first and third positions.

The mouthpiece cover is coupled to the dispensing mechanism such that movement of the mouthpiece cover drives the dispensing mechanism. In particular, the coupling is arranged such that movement of the mouthpiece cover from the first position to the second position does not causes the next medicament dose of the medicament carrier to move, and movement of the mouthpiece cover from the second position to the third position does cause the next medicament dose of the medicament carrier to be moved to the dispensing position, with the next medicament dose starting to move to the dispensing position when the mouthpiece cover moves through the second position.

Furthermore, according to this aspect of the invention, movement of the mouthpiece cover from the first position to the second position encloses an angle about the rotation axis of at least 5 degrees, and the mouthpiece component is completely covered when the mouthpiece cover is in the second position. By configuring the inhaler in this way, in particular such that movement of the mouthpiece cover from the first position to the second position defines an angle of at least 5 degrees, and such that the mouthpiece component is completely covered when the mouthpiece cover is in the second position, it results that even if the mouthpiece cover is unintentionally or accidentally moved between the first and second positions, there is no actuation of the dispensing mechanism, and also no risk of the mouthpiece component being exposed and/or contaminated. In particular, the mouthpiece component is not at risk of contamination during movement from the first position to the second position because the mouthpiece component is fully covered in both positions, and in between these positions. The dispensing mechanism is only actuated by moving the mouthpiece cover from the second position to a third position, in which the mouthpiece component is fully uncovered.

While the mouthpiece component cannot now be cleaned with the mouthpiece cover in the second position, it can still be cleaned when in the third position, for example immediately after use of the inhaler. This is not something that would have been apparent before the invention was made, owing to the fact that the prior art focuses on cleaning inhalers before use. Moreover, the inventors have recognised that routine cleaning of this type of inhaler is not generally required, and even less so because according to the invention the mouthpiece component is not as susceptible to contamination.

Movement of the mouthpiece cover from the first position to the second position may enclose an angle about the rotation axis of from 5 to 30 degrees, preferably from 5 to 15 degrees, more preferably from 8 to 15 degrees. In this way, the inhaler allows for significant unintentional or accidental movement of the mouthpiece cover when the inhaler is not in use, and undesired actuation of the dispensing mechanism or exposure of the mouthpiece component does not take place. In addition, there is still a further significant angle of movement between the second and third positions of the mouthpiece cover over which the dispensing mechanism can be progressively actuated, for example at least 70 degrees, and in specific embodiments 72 degrees or 90 degrees.

Movement of the mouthpiece cover from the second position to a position at which the mouthpiece component starts to be exposed may enclose an angle about the rotation axis of at least 5 degrees, preferably from 5 to 30 degrees, more preferably from 5 to 15 degrees, even more preferably from 8 to 15 degrees.

The first position of the mouthpiece cover may be a fully closed position from which the mouthpiece cover is only able to rotate in one direction. The third position of the mouthpiece cover may be a fully opened position from which the mouthpiece cover is only able to rotate in an opposite direction.

In certain embodiments, movement of the mouthpiece cover causes movement of at least one resilient drive pawl, and the at least one resilient drive pawl selectively engages a toothed drive gear of the dispensing mechanism for driving the dispensing mechanism to move the next medicament dose of the medicament carrier to the dispensing position. In this case, the at least one resilient drive pawl may be attached to or directly driven by the mouthpiece cover. The teeth of the drive gear may be ratchet teeth.

**In** particular, the inhaler may be arranged such that, on movement of the mouthpiece cover from the first position to the second position, the at least one resilient drive pawl is not engaged with any teeth of the drive gear (i.e. they are spaced-apart/separated), and, on movement of the mouthpiece cover from the second position to the third position, the at least one resilient drive pawl is engaged with (i.e. contacts and drives) at least one tooth of the drive gear.

The at least one resilient drive pawl described above may be provided about a first gear wheel which is rotatably driven by the mouthpiece cover. The drive gear described above may be provided as ratchet teeth about a second gear wheel arranged to rotate coaxially with the first gear wheel. In this case, the at least one resilient drive pawl may be provided about the outside of the first gear wheel and the drive gear may be provided as an internal gear about the second gear wheel.

The first gear wheel may be configured to rotate forwards and backwards when the mouthpiece cover is opened and closed, respectively, with the second gear wheel being configured to rotate forwards when driven forwards by the first gear wheel and not to rotate backwards, e.g. when the mouthpiece cover is closed. The second gear wheel may be provided with a ratchet and pawl arrangement for this purpose, i.e. for preventing reverse rotation. The teeth of the second gear wheel may be configured as ratchet teeth so that the resilient pawls of the first gear wheel are able to slide back over them when the first gear wheel rotates backwards.

The first and second gear wheels are preferably provided with a plurality of the resilient pawls and teeth, respectively, at evenly spaced and matching angular intervals. **In** a specific embodiment, the first gear wheel is provided with four resilient pawls at 90 degree intervals and the second gear wheel is provided with four teeth at 90 degree intervals. This arrangement allows for the first and second gear wheels to rotate through 90 degrees when the mouthpiece cover is moved from the second position to the third position, with the resilient pawls and teeth ending up with the same angular positions as they started. Such an arrangement facilitates resetting of the device after each use, since when the mouthpiece cover is subsequently closed, the resilient pawls and teeth are in the correct positions for the next use of the device. Providing exactly four evenly spaced pawls and four evenly spaced teeth therefore facilitates 90 degrees of travel when the mouthpiece cover is moved from the second position to the third position, which compared to a smaller angle of travel may result in a smaller force being needed to drive the dispensing mechanism.

In an another specific embodiment, the first gear wheel is provided with five resilient pawls at 72 degree intervals and the second gear wheel is provided with five teeth at 72 degree intervals. Such an arrangement allows for the first and second gear wheels to rotate through 72 degrees when the mouthpiece cover is moved from the second position to the third position, with the resilient pawls and teeth ending up with the same angular positions as they started. In embodiments, the mouthpiece cover may rotate through a total angle, from the first position to the third position, of at least 80 degrees.

It has been described above how, in some embodiments, movement of the mouthpiece cover causes movement of at least one resilient drive pawl, and the at least one resilient drive pawl then selectively engages a toothed drive gear of the dispensing mechanism. This arrangement may be reversed in other embodiments, so that movement of the mouthpiece cover causes movement of a toothed gear, and the toothed gear may selectively engage at least one resilient pawl of the dispensing mechanism for driving the dispensing mechanism. In this case, features relating to the resilient drive pawl described above may apply to the toothed gear, and vice versa.

According to a second aspect of the invention, there is provided dry powder inhaler for delivering medicament from at least one medicament carrier having a plurality of spaced-apart medicament doses, the dry powder inhaler comprising:
a housing having regions for accommodating unused and used portions of the medicament carrier;
a mouthpiece component joined to the housing and through which a user is able to inhale, the mouthpiece component including surfaces for contacting the user's lips;
a dispensing mechanism arranged in the housing for moving a next medicament dose of the medicament carrier to a dispensing position; and
a mouthpiece cover being rotatably connected to the housing for sequential movement about a rotation axis from a first position in which the mouthpiece component is at least partly covered to a second position, and from the second position to a third position in which the mouthpiece component is completely uncovered, the second position being between the first and third positions.

The mouthpiece cover is coupled to the dispensing mechanism such that movement of the mouthpiece cover drives the dispensing mechanism, with movement of the mouthpiece cover from the first position to the third position causing the next medicament dose of the medicament carrier to be moved to the dispensing position.

Furthermore, according to this aspect of the invention, the coupling of the mouthpiece cover to the dispensing mechanism is arranged to be non-linear, such that movement of the mouthpiece cover from the first position to the second position causes the next medicament dose to be continuously moved to an intermediate position with a first movement ratio, and movement of the mouthpiece cover from the second position to the third position causes the next medicament dose to be continuously moved from the intermediate position to the dispensing position with a second movement ratio which is different to the first movement ratio.

As used herein, the term "continuously moved" means that the next medicament dose moves in synchronisation with the mouthpiece cover, so that the next medicament dose moves at the same time as the mouthpiece cover (i.e. from the first position to the second position and from the second position to the third position).

This arrangement allows for improved operation of the inhaler, in particular for opening the mouthpiece cover for driving the dispensing mechanism to be made more adaptable. According to the invention, the rate at which the dispensing mechanism is driven when opening the mouthpiece cover (i.e. the ratio between movement of the next medicament dose and movement of the mouthpiece cover, referred to herein as a "movement ratio") can be varied so that there is no sudden increase or decrease in the force needed to operate the inhaler.

The first movement ratio is calculated as an average over the angle enclosed between the first and second positions of the mouthpiece cover, and the second movement ratio is calculated as an average over the angle enclosed between the second and third positions of the mouthpiece cover.

In this way, the inhaler can be made easier to use, and the elimination of sudden increases or decreases in force needed to move the mouthpiece cover as the dispensing mechanism starts to be driven or finishes being driven is perceived by the user as characteristic of a high quality, well-engineered device. It also helps to protect the mouthpiece cover and components of the dispensing mechanism from damage by avoiding excessive stress, and the resulting smoother operation helps to avoid vibration-induced movement of the powder medicament, which could otherwise result in poor dosing consistency and contamination of the internal components of the inhaler.

In some embodiments, the mouthpiece component may be completely covered when the mouthpiece cover is in the first position, in which case the mouthpiece component may be completely covered or partly covered when the mouthpiece cover is in the second position. In other embodiments, the mouthpiece component may be partly covered when the mouthpiece cover is in the first position, in which case the mouthpiece component may also be partly covered when the mouthpiece cover is in the second position.

The first position of the mouthpiece cover may be a fully closed position from which the mouthpiece cover is only able to rotate in one direction. The third position of the mouthpiece cover may be a fully opened position from which the mouthpiece cover is only able to rotate in an opposite direction. Movement from the first position to the second position may enclose an angle about the rotation axis of from 20 to 160 degrees, preferably from 30 to 90 degrees, more preferably from 35 to 60, and movement from the second position to the third position may enclose an angle about the rotation axis of from 20 to 160 degrees, preferably from 30 to 90 degrees, more preferably from 35 to 60 degrees.

**In** certain embodiments, the second movement ratio is greater than the first movement ratio, for example at least 5% greater, or at least 20% greater, or at least 40% greater, than the first movement ratio. The second movement ratio may be up to five times greater than the first movement ratio. An advantage of such an arrangement is that the dispensing mechanism is initially driven at a slow rate (i.e. low first movement ratio), which then increases to a faster rate (i.e. high second movement ratio). **In** this way, the force required to operate the inhaler may increase more progressively, as compared to known designs.

A further advantage of these embodiments is that by initially driving the dispensing mechanism at a lower rate (i.e. movement ratio), it is more likely that any unintentional or accidental actuation caused by movement of the mouthpiece cover between the first and second positions will be reversible. For example, where the dispensing mechanism is configured to peel open or pierce a medicament-containing blister towards the end of its actuation cycle, it is more likely that the unintentional or accidental movement will not have caused the blister to be peeled or pierced.

**In** some embodiments, the mouthpiece cover may drive a first gear wheel and the dispensing mechanism is driven by a second gear wheel, and the first and second gear wheels form a gear train that is in meshed engagement at least during opening of the mouthpiece cover.

**In** certain embodiments, the first and second gear wheels have respective first segments having first pitch circle diameters that define a first gear ratio corresponding to the first movement ratio, and the first and second gear wheels have respective second segments having second pitch circle diameters that define a second gear ratio corresponding to the second movement ratio. Such an arrangement provides a step change in the gearing between the mouthpiece cover and the dispensing mechanism.

In other embodiments, the first and second gear wheels have respective segments with pitch circle diameters that vary, without step changes, such that a gear ratio between the first and second gear wheels varies, without step changes. Such an arrangement provides a gradual change in the gearing between the mouthpiece cover and the dispensing mechanism. Such an arrangement may allow for especially smooth driving of the dispensing mechanism.

In alternative embodiments, the first and second gear wheels may form a Geneva drive. In particular, the first gear wheel may carry a pin of the Geneva drive and the second gear wheel may be provided with a plurality of slots of the Geneva drive for engagement by the pin. Such an arrangement allows for smooth driving of the dispensing mechanism.

In other embodiments, the mouthpiece cover drives a rotatable cam and the dispensing mechanism is driven by a follower. The cam engages the follower, such that movement of the mouthpiece cover from the first position to the third position causes the dispensing mechanism to be driven with a gear ratio that varies, without any step changes in the gear ratio.

In other embodiments, the mouthpiece cover includes an eccentrically mounted pin, and the dispensing mechanism is driven by a gear wheel or rotatable linkage having a radially-extending slot for receiving the pin. During opening of the mouthpiece cover, the pin is arranged to engage the slot to thereby rotate the gear wheel, such that movement of the mouthpiece cover from the first position to the third position causes the dispensing mechanism to be driven with a gear ratio that varies, without any step changes in the gear ratio.

In other embodiments, the dispensing mechanism is driven by a gear wheel, a hub associated with the gear wheel being rotationally driven by the opening of the mouthpiece cover. The hub and the gear wheel are resiliently coupled, such that movement of the mouthpiece cover from the first position to the second position causes torsional deformation of the resilient coupling along with movement of the next medicament dose with the first movement ratio, and movement of the mouthpiece cover from the second position to the third position causes a lesser amount of (or no) torsional deformation of the resilient coupling along with movement of the next medicament dose with the second movement ratio. During movement of the mouthpiece cover from the second position to the third position, the hub may directly engage the gear wheel so that there is no further torsional deformation of the resilient coupling. Movement of the mouthpiece cover from the first position to the third position may cause the dispensing mechanism to be driven with a gear ratio that varies, without any step changes in the gear ratio.

In the embodiments described above, a gear ratio may (gradually) increase from the first position to the third position of the mouthpiece cover.

Furthermore, in general, the dispensing mechanism may comprises an anti-reverse rotation mechanism, such that movement of the mouthpiece cover from the first position to the third position causes the next medicament dose to be moved, and movement of the mouthpiece cover from the third position to the first position does not causes the next medicament dose to be moved. The anti-reverse rotation mechanism may comprise a ratchet and pawl arrangement.

In general, and applicable to all aspects of the invention, the dry powder inhaler may further comprise a manifold arranged in the housing, the manifold defining at least part of an air flow path extending from an air inlet to the mouthpiece component, and the manifold also defining the dispensing position for the next medicament dose of the medicament carrier.

The dispensing mechanism may be configured to place the next medicament dose at the dispensing position in fluid communication with the air flow path, ready for inhalation by the user, for example by selectively peeling or piercing a portion of the medicament carrier.

In any of the above-described embodiments, the medicament carrier may comprise an elongate blister strip having a base layer defining spaced-apart blister openings containing medicament doses, and a cover layer adhesively bonded to the base layer to close the blisters, and wherein the cover layer is arranged to be peeled from the base layer.

The dispensing mechanism may then comprise: an indexing mechanism for moving a blister of the elongate blister strip to the dispensing position; and a peeling mechanism for peeling the cover layer from the blister for placing the next medicament dose in fluid communication with the air flow path ready for inhalation by the user.

The dry powder inhaler may further comprise the medicament carrier, for example a single medicament carrier, which may for example be arranged in a replaceable cartridge received by the housing. The spaced-apart medicament doses may comprise essentially any inhalable powder medicament, for example medicaments for the treatment of respiratory disorders such as asthma, chronic obstructive pulmonary disease (COPD), bronchitis and chest infections. Suitable powder medicaments may include for example any one of long acting beta antagonists (LABA) , short acting beta antagonists (SABA), corticosteroids (ICS), long acting muscarinic antagonists (LAMA), short acting muscarinic antagonists (SAMA), or any other drug that can be administered via inhalation including combination thereof. Examples for such drugs include but are not limited to budesonide, formoterol, beclomethasone, fluticasone, salmeterol, albuterol, salbutamol, indacaterol, tiotropium, ipratropium, glycorpyrronium , umeclidinium, vilanterol and combination thereof. Where fluticasone is mentioned, this term is intended to cover any suitable ester form, in particular fluticasone propionate or fluticasone furoate.

In embodiments, the indexing mechanism may be arranged to move at least two blisters of the elongate blister strip to the dispensing position at a time, and the peeling mechanism may be arranged to peel the cover layer from the at least two blisters for placing at least two medicament doses in fluid communication with the air flow path ready for inhalation by the user. In this way, the dry powder inhaler can be used for simultaneous inhalation of different medicaments from different blisters.

The dry powder inhaler may further comprise the medicament carrier, for example a single medicament carrier, which may be arranged in a replaceable cartridge received by the housing.

In this case, the different medicaments for simultaneous inhalation may comprise: LABA or SABA in a first blister and an ICS in a second blister, LABA or SABA in a first blister and LAMA or SAMA in a second blister, LABA or SABA and LAMA or SAMA in a first blister and ICS in a second blister, for example budesonide in a first blister and formoterol in a second blister; or beclomethasone in a first blister and formoterol in a second blister; or fluticasone in a first blister and salmeterol in a second blister, or fluticasone in a first blister and albuterol in a second blister, or fluticasone in a first blister and vilanterol in a second blister, or umeclidinium in a first blister and vilanterol in a second blister, or two selected from umeclidinium, fluticasone and vilanterol in a first blister, the remaining medicament from umeclidinium, fluticasone and vilanterol in a second blister.

Alternatively, the dry powder inhaler may be arranged for delivering medicaments from a plurality of medicament carriers each having a plurality of spaced-apart medicament doses. Each of the medicament carriers may comprise an elongate blister strip having a base layer defining spaced-apart blister openings containing medicament doses, and a cover layer adhesively bonded to the base layer to close the blisters, and wherein the cover layer is arranged to be peeled from the base layer.

In this case, the dispensing mechanism may comprise: an indexing mechanism for moving a blister of each of the elongate blister strips to the dispensing position; and a peeling mechanism for peeling the cover layer from each of the blister strips for placing the medicament doses in fluid communication with the air flow path ready for simultaneous inhalation by the user.

The dry powder inhaler may further comprise the medicament carriers, for example exactly two medicament carriers, which may be arranged in at least one replaceable cartridge received by the housing. The medicament carriers may comprise respective different medicaments for simultaneous inhalation. The different medicaments may comprise: LABA or SABA in a first medicament carrier and an ICS in a second medicament carrier, LABA or SABA in a first medicament carrier and LAMA or SAMA in a second medicament carrier, LABA or SABA and LAMA or SAMA in a first medicament carrier and ICS in a second medicament carrier, for example budesonide in a first medicament carrier and formoterol in a second medicament carrier; or beclomethasone in a first medicament carrier and formoterol in a second medicament carrier; or fluticasone in a first medicament carrier and salmeterol in a second medicament carrier, or fluticasone in a first medicament carrier and albuterol in a second medicament carrier, or fluticasone in a first medicament carrier and vilanterol in a second medicament carrier, or umeclidinium in a first medicament carrier and vilanterol in a second medicament carrier, or two selected from umeclidinium, fluticasone and vilanterol in a first medicament carrier, the remaining medicament from umeclidinium, fluticasone and vilanterol in a second medicament carrier.

### BRIEF DESCRIPTION OF THE DRAWINGS

Embodiments of the invention will now be described in more detail and by way of non-limiting examples with reference to the accompanying drawings, in which:
Fig. 1 is a perspective view of a dry powder inhaler according to a first embodiment of the invention;
Fig. 2 is a perspective view of a medicament carrier for use in the dry powder inhaler shown in Fig. 1;
Fig. 3 is a view of the inhaler shown in Fig. 1 with certain components removed and including a pair of the medicament carriers shown in Fig. 2 installed;
Fig. 4 is an exploded perspective view of the inhaler shown in Fig. 1 with certain components removed, from the opposite direction;
Figs. 5a and 5b are schematic views showing the inhaler and a part of its dispensing mechanism in a first position of the mouthpiece cover;
Figs. 6a and 6b are schematic views showing the inhaler and a part of its dispensing mechanism in a second position of the mouthpiece cover;
Figs. 7a and 7b are schematic views showing the inhaler and a part of its dispensing mechanism in a third position of the mouthpiece cover;
Fig. 8 is a graphical representation of operation of the dispensing mechanism of the dry powder inhaler shown in Fig. 1;
Figs. 9a to 9c are schematic views showing an inhaler according to a second embodiment of the invention with its mouthpiece cover in first, second and third positions;
Figs. 10a to 10c are schematic views showing a non-linear transmission means of the inhaler when the mouthpiece cover is in the first, second and third positions;
Fig. 11 is a graphical representation of operation of the non-linear transmission means shown in Figs. 10a to 10c;
Figs. 12a to 12c are schematic views showing a non-linear transmission means of a first alternative embodiment when the mouthpiece cover is in the first, second and third positions;
Fig. 13 is a graphical representation of operation of the non-linear transmission means shown in Figs. 12a to 12c;
Figs. 14a to 14c are schematic views showing a non-linear transmission means of a second alternative embodiment when the mouthpiece cover is in the first, second and third positions;
Fig. 15 is a graphical representation of operation of the non-linear transmission means shown in Figs. 14a to 14c;
Figs. 16a to 16c are schematic views showing a non-linear transmission means of a third alternative embodiment when the mouthpiece cover is in the first, second and third positions;
Fig. 17 is a graphical representation of operation of the non-linear transmission means shown in Figs. 16a to 16c;
Figs. 18a to 18c are schematic views showing another non-linear transmission means that may be used in the inhaler according to the first or second embodiment;
Fig. 19 is an exemplary graphical representation of operation of the non-linear transmission means shown in Figs. 18a to 18c;
Figs. 20a to 20c are schematic views showing yet another non-linear transmission means that may be used in the inhaler according to the first or second embodiment;
Fig. 21 is an exemplary graphical representation of operation of the non-linear transmission means shown in Figs. 20a to 20c; and
Fig. 22 is a schematic view showing a transmission means that may be used in the inhaler according to the first embodiment.

### DETAILED DESCRIPTION OF THE EMBODIMENTS

It should be understood that the detailed description, while indicating exemplary embodiments of the inventive dry powder inhaler, are intended for the purposes of illustration only and are not intended to limit the scope of the invention. Features, aspects, and advantages of the inhaler will become better understood from the following description, appended claims, and accompanying drawings. It should be understood that the Figures are merely schematic and are not drawn to scale. It should also be understood that the same reference numerals are used throughout the Figures to indicate the same or similar parts.

The first aspect of the invention provides a dry powder inhaler for delivering medicament from at least one medicament carrier having a plurality of spaced-apart medicament doses. The inhaler comprises: a housing having regions for accommodating unused and used portions of the medicament carrier; a mouthpiece component joined to the housing and through which a user is able to inhale; a manifold arranged in the housing and defining at least part of an air flow path extending from an air inlet to the mouthpiece, the manifold further defining a dispensing position for the medicament carrier; a dispensing mechanism arranged in the housing for moving a next medicament dose of the medicament carrier to the dispensing position and for placing the medicament dose in fluid communication with the air flow path ready for inhalation by the user; and a mouthpiece cover being rotatably connected to the housing for sequential movement about a rotation axis from a first position in which the mouthpiece component is completely covered to a second position in which the mouthpiece component is also completely covered, and from the second position to a third position in which the mouthpiece component is completely uncovered (the second position being between the first and third positions).

The mouthpiece cover is coupled to the dispensing mechanism such that movement of the mouthpiece cover drives the dispensing mechanism. In particular, movement of the mouthpiece cover from the first position to the second position does not causes the next medicament dose to be moved, and movement of the mouthpiece cover from the second position to the third position does cause the next medicament dose to be moved to the dispensing position, with the next medicament dose starting to move to the dispensing position when the mouthpiece cover moves through the second position.

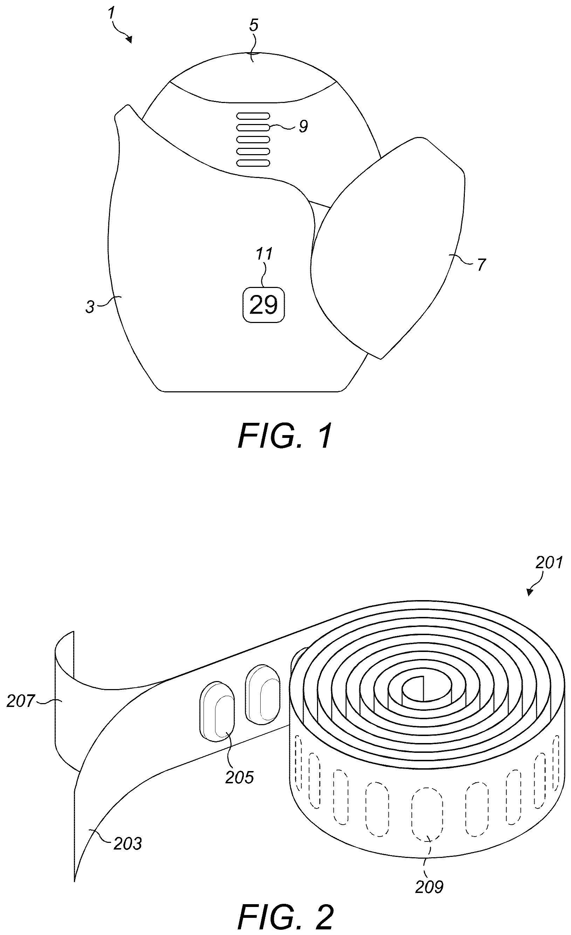

Figs. 1, 3 and 4 show a dry powder inhaler 1 according to a non-limiting embodiment of the first aspect of the invention. Fig. 2 shows a medicament carrier 201 for use with the inhaler 1. A dry powder inhaler using the same type of medicament carrier is described in WO 2007/012871 A1, the entire contents of which is incorporated herein by reference.

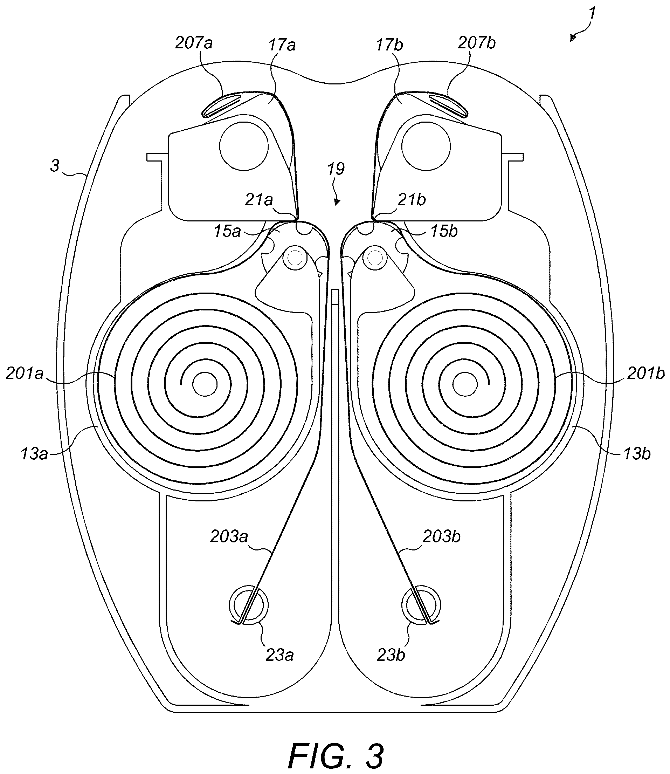

As shown in Fig. 1, the inhaler 1 is a hand-held device having a substantially flat or planar shape with a rounded outer profile. The outer profile of the inhaler is mainly defined by its housing 3 which encloses its internal components and receives at least one medicament carrier 201 (not shown in Fig. 1 but shown in Figs. 2 and 3). Extending from the housing 3 is a mouthpiece component 5 having a central opening through which the user inhales the powder medicament (the terms "mouthpiece component" and "mouthpiece" are used interchangeably herein to mean a mouthpiece component). The mouthpiece component 5 is assembled or joined to the housing 3.

The inhaler also comprises the mouthpiece cover 7, which is rotatably connected to the housing 3 for sequential movement about a rotation axis from a first position in which the mouthpiece 5 is completely covered to a second position in which the mouthpiece 5 is also completely covered, and from the second position to a third position in which the mouthpiece 5 is completely uncovered. Fig. 1 shows the mouthpiece cover 7 in its third position.

Also visible in Fig. 1 is an air inlet 9 and a dose counter display 11. The air inlet 9, which can be covered by the mouthpiece cover 7, defines the start of an air flow path that extends from the air inlet 9 to the central opening of the mouthpiece 5. The dose counter display 11 is coupled to a dispensing mechanism of the inhaler 1, to be described subsequently, so as to provide an indication of the number of medicament doses used or remaining in the inhaler 1.

Fig. 2 shows a medicament carrier 201 for use with the inhaler 1 shown in Fig. 1. The medicament carrier 201 is in the form of an elongate blister strip. The blister strip comprises a semi-rigid base layer 203 which is formed with spaced-apart blister openings 205 and more flexible cover layer 207 which covers the blister openings 205 to define spaced-apart blisters 209. The blisters 209 each contain a sealed dose of powder medicament.

The cover layer 207 of the medicament carrier 201 is adhesively bonded to the base layer 203 such that the layers 203, 207 can be peeled apart to open the blisters 209 and liberate the powder medicament without any risk of either layer 203, 207 breaking. The base layer 203 and the cover layer 207 typically comprise plastics/aluminium laminates and are adhesively bonded by a heat seal lacquer. A suitable medicament carrier is described in more detail in WO 2007/012871 A1.

The inhaler 1 shown in Fig. 1 is designed for use with a pair (i.e. two) of the medicament carriers 201 shown in Fig. 2, as will now be described with reference to Fig. 3. It is to be noted, however, that embodiments according to the invention may be used with, or comprise, any number of the medicament carriers 201, including just one.

Fig. 3 is a view of the inhaler 1 shown in Fig. 1 with certain components removed and including a pair of the medicament carriers 201a, 201b installed. In particular, the mouthpiece 5, mouthpiece cover 7, and part of the housing 3 have been removed to reveal a remaining part of the housing 3 and the medicament carriers 201a, 201b, as well as parts of the dispensing mechanism. As shown in the drawing, the housing 3 provides regions 13a, 13b for accommodating unused portions of the medicament carriers 201a, 201b, which are loosely-coiled. Regions are also provided for accommodating used portions of the medicament carriers 201a, 201b, as will be described below. It is noted that the internal arrangement of the inhaler 1 is generally symmetrical, with one medicament carrier 201a being accommodated on one side (left side in the drawing) and the other medicament carrier 201b being accommodated on the other side (right side in the drawing).

The dispensing mechanism, which is only partly shown in Fig. 3, comprises an indexing wheel 15a, 15b and a peeling spool 17a, 17b for each of the medicament carriers 201a, 201b. Each medicament carrier 201a, 201b is fed around its respective indexing wheel 15a, 15b with the base layer 203a, 203b having the blisters facing the indexing wheel 15a, 15b. The indexing wheels 15a, 15b are provided with a plurality of recesses about their circumference which are sized and positioned to receive the blisters of the medicament carrier 201a, 201b. When the index wheels 15a, 15b are rotatably driven by the dispensing mechanism, they engage the medicament carriers 201a, 201b, in particular their semi-rigid blisters, so that the next medicament-containing blister of each medicament carrier 201a, 201b can be advanced to a dispensing position 19 of the inhaler 1 (at which the medicament is to be presented for inhalation).

Simultaneously, a leading end of the cover layer 207a, 207b of each medicament carrier 201a, 201b is separated from its base layer 203a, 203b and fed around a peeling edge 21a, 21b, which is positioned between used portions of the cover layer 207a, 207b and the base layer 203a, 203b. For this purpose, the leading end of the cover layer 207a, 207b of each medicament carrier 201a, 201b is attached to a respective peeling spool 17a, 17b. The peeling spools 17a, 17b are rotatably driven at the same time as the indexing wheels 15a, 15b, and this causes each cover layer 207a, 207b to be gradually peeled away from its base layer 203a, 203b at the peeling edge 21a, 21b, so that the medicament-containing blister 209a, 209b is opened for inhalation by the user.

As medicament doses are dispensed from the inhaler 1, used portions of the cover layers 207a, 207b are wound onto the peeling spools 17a, 17b. Used portions of the base layers 203a, 203b are accommodated in a separate region of the housing where they are coiled up by rotatably-driven coiling spools 23a, 23b. By way of example, the medicament carriers 201a, 201b may each comprise 60 doses of a powder medicament, with a dose from each carrier being dispensed simultaneously.

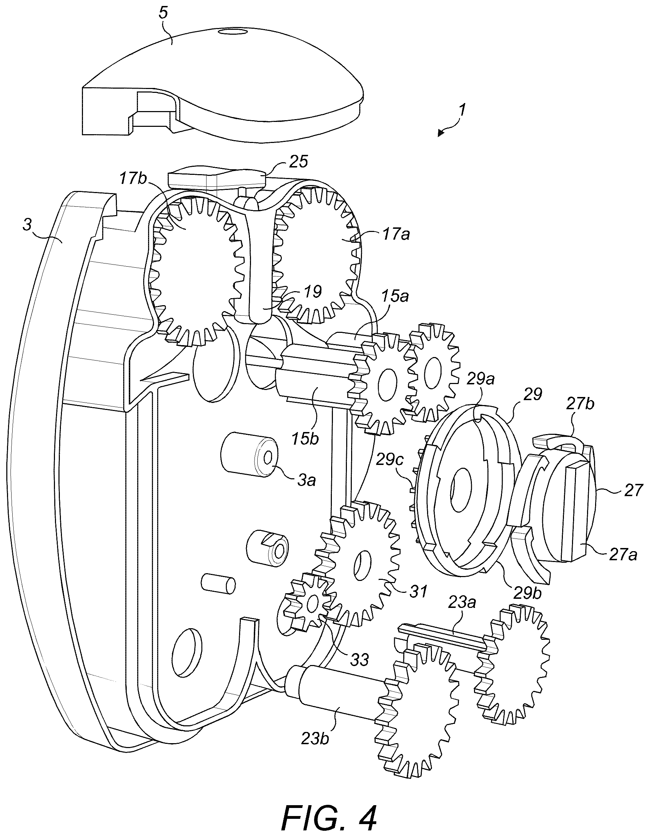

The remaining parts of the dispensing mechanism of the inhaler 1 will now be described with reference to Fig. 4, which is an exploded perspective view of the inhaler shown in Fig. 1 with certain components removed. It is noted that Fig. 4 shows an opposite side of the inhaler to that shown in Fig. 3, i.e. Fig. 4 shows the back side of the inhaler 1. In Fig. 4, the mouthpiece cover 7, and part of the housing 3 have been removed to reveal a remaining part of the housing 3 and remaining parts of the dispensing mechanism. Fig. 4 also shows the mouthpiece 5 and a manifold 25.

The manifold 25 is a moulded plastics component arranged in the housing 3 and which defines an air flow passage extending from the air inlet 9 (Fig. 1) to the central opening of the mouthpiece 5. When a user inhales through the mouthpiece 5, air is drawn in to the air inlet 9, through the air flow passage, and out through the mouthpiece 5. The manifold 25 is arranged in such a way that the air flow passage is provided with at least one opening adjacent to the dispensing position 19 of the inhaler, so that the medicament doses of opened blisters are placed in fluid communication with the air flow path. In this way, when the user inhales through the mouthpiece 5, the powder medicament can be drawn out of the blisters by the turbulent airflow and inhaled.

Fig. 4 shows the back sides of the indexing wheels 15a, 15b, peeling spools 17a, 17b and coiling spools 23a, 23b, each of which is rotatably mounted in the housing and provided with a gear wheel for driven rotation. The gear wheels form part of a gear train which is driven by opening the mouthpiece cover 7 (Fig. 1).

For this purpose, the mouthpiece cover 7 (not shown in Fig. 4) is coupled to a first gear wheel 27, which is arranged to rotate with the mouthpiece cover 7 about its rotation axis. In particular, the first gear wheel 27 is arranged to rotate forwards and backwards when the mouthpiece cover 7 is opened and closed.

The first gear wheel 27 is arranged to selectively drive a second gear wheel 29 which is mounted coaxially with the first gear wheel 27 on a stub shaft 3a. The second gear wheel 29 is a drive gear of the dispensing mechanism and directly or indirectly drives the indexing wheels 15a, 15b, peeling spools 17a, 17b and coiling spools 23a, 23b when the mouthpiece cover 7 is opened. The coiling spools 23a, 23b are also driven via idler gear wheels 31, 33. The first and second gear wheels 27, 29 will now be described in greater detail.

The first gear wheel 27 is directly coupled to a hub of the mouthpiece cover 7, which is covered by the housing 3 in Fig. 1. For this purpose, a front face of the first gear wheel 27 is provided with a raised rib 27a, which is received into a corresponding slot of the hub. When the mouthpiece cover 7 is opened and closed, the slot engages the rib 27a to transmit torque, so that the first gear wheel 27 rotates with the mouthpiece cover 7.

The outer circumference of the first gear wheel 27 is provided with a plurality, in this embodiment five, resilient drive pawls 27b which are equally spaced-apart at an angle of 72 degrees. **It** should be noted that other embodiments may comprise a different number of resilient drive pawls 27b. For example, another preferred embodiment comprises four resilient drive pawls which are equally spaced-apart at an angle of 90 degrees.

The resilient drive pawls 27b of the first gear wheel 27 are arranged to selectively engage corresponding ratchet teeth 29a formed as an internal gear on the second gear wheel 29. In particular, when the first gear 27 is rotated forwards upon opening of the mouthpiece cover 7, the resilient drive pawls 27b come into contact with and engage the ratchet teeth 29a of the second gear wheel 29 so as to drive it forwards. However, when the first gear 27 is rotated backwards upon closing of the mouthpiece cover 7, the resilient drive pawls 27b slide over the ratchet teeth 29a of the second gear wheel 29 so that it is not driven.

The second gear wheel 29 is provided about its outer circumference with a second plurality of ratchet teeth 29b which function, together with a fixed pawl attached to the housing (not shown), as a means for preventing reverse rotation of the second gear wheel 29.

The second gear wheel 29 is also provided about its outer circumference, in a different axial plane, with a set of ordinary gear teeth 29c which engage and drive a first one of the indexing wheels 15b. This indexing wheel 15b drives the other indexing wheel 15a and a first one of the peeling spools 17b. The other indexing wheel 15a drives the other peeling spool 17a. It will be understood that the gear train is sequenced so as to ensure that each of the driven elements rotates in the appropriate direction for dispensing the powder medicament from the blisters of the medicament carriers 201a, 201b.

Further detail relating to the structure and design of a suitable dispensing mechanism can be found for example in WO 2007/012871 A1.

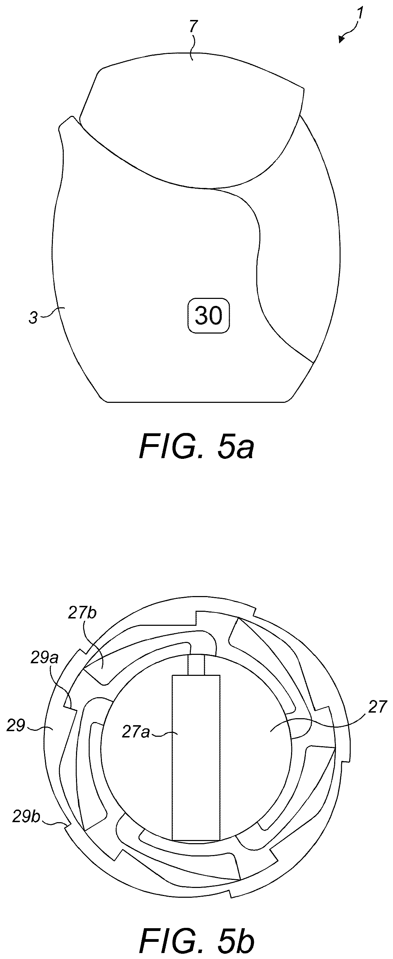

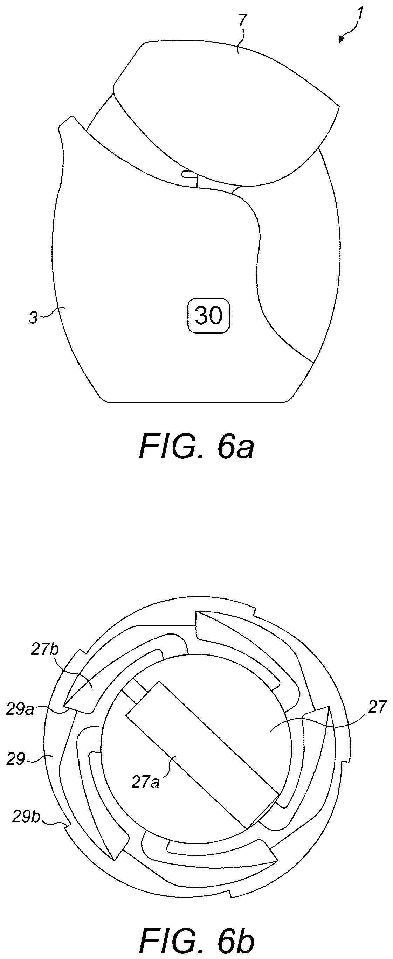

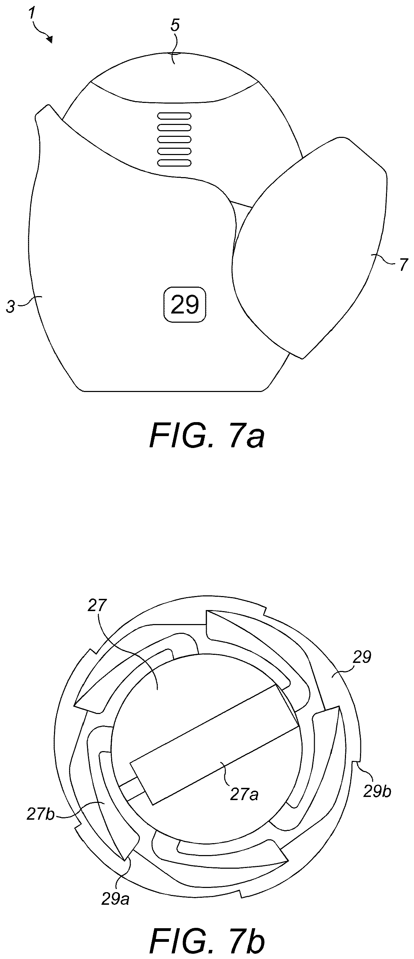

Operation of the inhaler and, in particular of the mouthpiece cover 7 and dispensing mechanism, will now be described with reference to Figs. 5a to 7b. Figs. 5a and 5b are schematic views showing the inhaler 1 and a part of its dispensing mechanism in a first position of the mouthpiece cover 7. Figs. 6a and 6b are schematic views showing the inhaler 1 and a part of its dispensing mechanism in a second position of the mouthpiece cover 7. Figs. 7a and 7b are schematic views showing the inhaler 1 and a part of its dispensing mechanism in a third position of the mouthpiece cover 7. It is noted that Figs. 5b, 6b and 7b show an opposite (back) side of the inhaler to that shown in Figs. 5a, 6a and 7a (which is a front side).

Referring now to Fig. 5a, the inhaler 1 is shown with the mouthpiece cover 7 in its first position, which is its normal (completely closed) position when the inhaler 1 is not in use. In this position, the mouthpiece 5 is completely covered by the mouthpiece cover 7 and the mouthpiece cover 7 is rotated to the maximum extent possible in the counter-clockwise direction (also referred to herein as the backwards direction). In this position, the mouthpiece 5 is completely protected from contamination. Such contamination can be unhygienic and can potentially affect the medicament delivery performance of the inhaler 1.

Fig. 5b is a view showing the first gear wheel 27 and second gear wheel 29 when the mouthpiece cover 7 is in the first position shown in Fig. 5a. In this position, the resilient drive pawls 27b of the first gear wheel 27 are spaced away from, and are not therefore engaged with, the corresponding ratchet teeth 29a formed as an internal gear on the second gear wheel 29.

Fig. 6a shows the inhaler 1 after the mouthpiece cover 7 has been moved from the first position to the second position. In this position, the mouthpiece cover 7 has rotated clockwise (or forwards) but the mouthpiece 5 is still completely covered by the mouthpiece cover 7. Movement of the mouthpiece cover 7 from the first position to the second position may enclose an angle of at least 5 degrees and, in the illustrated embodiment, encloses an angle of 10 degrees.

Fig. 6b is a view showing the first gear wheel 27 and second gear wheel 29 when the mouthpiece cover 7 is in the second position shown in Fig. 6a. In this position, the first gear wheel 27 has rotated forwards (counter-clockwise in this view) to bring the resilient drive pawls 27b just into contact with the corresponding ratchet teeth 29a of the second gear wheel 29. The second gear wheel 29 has not, however, moved, and so the dispensing mechanism of the inhaler 1 has not yet been driven.

Fig. 7a shows the inhaler 1 after the mouthpiece cover 7 has been moved from the second position to the third position. In this position, the mouthpiece cover 7 has rotated further clockwise (or forwards) to completely uncover the mouthpiece 5 ready for the user to inhale dispensed doses of the powder medicament. In this position, the mouthpiece cover 7 is rotated to the maximum extent possible in the clockwise direction. For reasons that will become apparent from the following description, movement of the mouthpiece cover 7 from the second position to the third position encloses an angle of exactly 72 degrees. This implies that, in the illustrated embodiment, movement of the mouthpiece cover 7 from the first position to the third position encloses a total angle of 82 degrees (i.e. 10 degrees plus 72 degrees).

Fig. 7b is a view showing the first gear wheel 27 and second gear wheel 29 when the mouthpiece cover 7 is in the third position shown in Fig. 7a. In this position, the first gear wheel 27 has rotated further forwards (counter-clockwise in this view) and in this case the resilient drive pawls 27b have engaged the corresponding ratchet teeth 29a of the second gear wheel 29 to drive the second gear wheel 29 forwards. This driving of the second gear wheel 29 functions to drive the dispensing mechanism so that the next medicament-containing blister of each medicament carrier 201a, 201b is moved to the dispensing position 19 of the inhaler 1 and so that the cover layers 207a, 207b are peeled away to place the medicament doses in fluid communication with the air flow passage of the manifold 25, ready for inhalation by the user.

The mouthpiece cover 7 is configured so that movement from the second position to the third position encloses an angle of exactly 72 degrees because this results in the five resilient drive pawls 27b of the first gear wheel 27 and the corresponding five ratchet teeth 29a of the second gear wheel 29 rotating through 72 degrees so that they start and end with the same angular positions, as can be seen by comparing Figs. 6b and 7b, which facilitates resetting the device when the mouthpiece cover 7 is subsequently closed.

In particular, when the mouthpiece cover 7 is closed after use of the inhaler 1, by rotating the mouthpiece cover 7 counter-clockwise from the third position shown in Fig. 7a to the first position shown in Fig. 5a, the second gear wheel 29 is prevented from rotating backwards (clockwise in the views of Figs. 5b, 6b and 7b) owing to the means for preventing reverse rotation 29b and therefore remains in the same angular position. The first gear wheel 27 rotates backwards (clockwise in the views of Figs. 5b, 6b and 7b) as the mouthpiece cover 7 is closed, with the resilient drive pawls 27b sliding back over the ratchet teeth 29a of the second gear wheel 29 to the position shown in Fig. 6b and then back to the position shown in Fig. 5b.

With the mouthpiece cover 7 closed, the five resilient drive pawls 27a of the first gear wheel 27 and the corresponding five ratchet teeth 29a of the second gear wheel 29 have the positions shown in Fig. 5b, ready for the next use.

The inhaler 1 described above comprises a first gear wheel 27 having five resilient drive pawls 27b. It should be noted that the first gear wheel may instead comprise a different number of resilient drive pawls. For example, the first gear wheel may be provided with four resilient drive pawls equally spaced-apart at 90 degree intervals (and correspondingly the second gear wheel may be provided with four ratchet teeth equally spaced-apart at 90 degree intervals), in which case movement of the mouthpiece cover from the second position to the third position would encloses an angle of exactly 90 degrees (360 degrees divided by four).

The inhaler 1 described above provides an arrangement in which movement of the mouthpiece cover 7 from the first position shown in Fig. 5a to the second position shown in Fig. 6a does not result in any actuation of the dispensing mechanism and does not uncover the mouthpiece 5, even partly. As such, actuation caused by unintentional or accidental movement of the mouthpiece cover when the inhaler is not in use can be avoided. Further, such unintentional or accidental movement of the mouthpiece cover does not risk of contamination of the mouthpiece, since the mouthpiece remains completely covered.

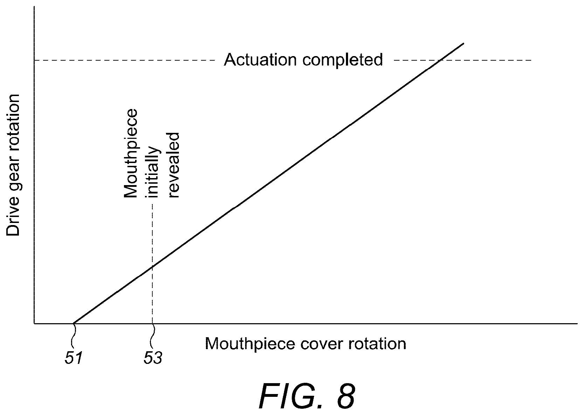

Fig. 8 provides a graphical representation of this operation of the dispensing mechanism. In particular, Fig. 8 illustrates how rotation of the mouthpiece cover is translated into actuation (driving) of the dispensing mechanism. It can be seen that initial opening of the mouthpiece cover from the first position to the second position 51 does not actuate the dispensing mechanism. Further rotation of the mouthpiece cover from the second position 51 to the third position 53 causes full actuation of the dispensing mechanism.

The second aspect of the invention will now be described in detail. The second aspect of the invention provides a dry powder inhaler for delivering medicament from at least one medicament carrier having a plurality of spaced-apart medicament doses, the dry powder inhaler comprising: a housing having regions for accommodating unused and used portions of the medicament carrier; a mouthpiece component joined to the housing and through which a user is able to inhale; a manifold arranged in the housing and defining at least part of an air flow path extending from an air inlet to the mouthpiece, the manifold further defining a dispensing position for the medicament carrier; a dispensing mechanism arranged in the housing for moving a next medicament dose of the medicament carrier to the dispensing position and for placing the medicament dose in fluid communication with the air flow path ready for inhalation by the user; and a mouthpiece cover being rotatably connected to the housing for sequential movement about a rotation axis from a first position in which the mouthpiece component is at least partly covered to a second position, and from the second position to a third position in which the mouthpiece component is completely uncovered, the second position being between the first and third positions.

The mouthpiece cover is coupled to the dispensing mechanism such that movement of the mouthpiece cover drives the dispensing mechanism. In particular, movement of the mouthpiece cover from the first position to the third position causes the next medicament dose of the medicament carrier to be moved to the dispensing position.

According to the second aspect, the coupling of the mouthpiece cover to the dispensing mechanism is arranged to be non-linear, such that movement of the mouthpiece cover from the first position to the second position causes the next medicament dose to be continuously moved to an intermediate position with a first movement ratio, and movement of the mouthpiece cover from the second position to the third position causes the next medicament dose to be continuously moved from the intermediate position to the dispensing position with a second movement ratio which is different to the first movement ratio.

As noted hereinabove, the term "movement ratio" refers to the ratio between movement of the medicament dose and movement of the mouthpiece cover, for example the ratio between linear movement (displacement) of the medicament dose as the medicament carrier is fed through the dispensing mechanism and angular movement of the mouthpiece cover. The first movement ratio is calculated as an average over the angle defined between the first and second positions of the mouthpiece cover. The second movement ratio is calculated as an average over the angle defined between the second and third positions of the mouthpiece cover. These average movement ratios may be calculated as a ratio of the displacements (linear or angular displacement of the medicament dose and angular displacement of the mouthpiece cover).

A non-limiting embodiment of the second aspect of the invention will now be described with reference to the drawings. The inhaler 1 of the second aspect has a structure that is identical to that shown in Figs. 1 to 3, and the following description will therefore focus on those parts of the inhaler 1 that are different, in particular with reference to Figs. 4 and 9a to 11.

Thus, in brief summary, the inhaler 1 of the second aspect comprises the housing 3, with the mouthpiece component 5 and the mouthpiece cover 7 shown in Fig. 1. The inhaler 1 is configured for use with a pair of the medicament carriers 201 shown in Fig. 2, and these are received by the inhaler 1 in the manner shown in Fig. 3. The inhaler 1 has the dispensing mechanism comprising pairs of indexing wheels 15a, 15b, peeling spools 17a, 17b and coiling spools 23a, 23b, as illustrated in Fig. 3. For further detail, reference is made to the description of Figs. 1 to 3 set out above in relation to the inhaler of the first aspect, all of which is applicable to the inhaler of the second aspect.

The remaining parts of the dispensing mechanism of the inhaler 1 of the second aspect will now be described with reference to Figs. 4 and 9a to 11.

The dispensing mechanism of the inhaler 1 of the second aspect is substantially the same as is shown in Fig. 4. Thus, the inhaler has the mouthpiece 5 and the manifold 25, as shown and described hereinabove. Further, the indexing wheels 15a, 15b, peeling spools 17a, 17b and coiling spools 23a, 23b of the dispensing mechanism are each rotatably mounted in the housing and provided with a gear wheel for driven rotation, in the manner shown in Fig. 4. These gear wheels form part of a gear train which is driven by opening the mouthpiece cover 7 (Fig. 1).

For this purpose, the mouthpiece cover 7 (not shown in Fig. 4) is arranged to drive the first gear wheel 27, and the first gear wheel 27 is arranged to selectively drive a second gear wheel 29, which functions as the drive gear of the dispensing mechanism. It is the particular manner in which the mouthpiece cover 7 is coupled to the first gear wheel 27, and the particular design of the first and second gear wheels 27, 29, that distinguishes the inhaler 1 of the second aspect from what is shown in Fig. 4.

In the inhaler 1 of the second aspect, the mouthpiece cover 7 is reversibly coupled to the first gear wheel 27 so that opening of the mouthpiece cover 7 causes the first gear wheel 27 to rotate in one direction, and so that closing of the mouthpiece cover 7 causes the first gear wheel 27 to rotate in the other direction. The coupling takes the form of a non-linear transmission means, to be described below.

The first and second gear wheels 27, 29 of the inhaler 1 of this aspect are similar to those shown in Fig. 4, but are configured to function as a simple one-way clutch, such that all rotation of the first gear wheel 27 when the mouthpiece cover 7 is opened is transmitted to the second gear wheel 29, and any rotation of the first gear wheel 27 when the mouthpiece cover 7 is closed is not transmitted to the second gear wheel 29. By way of example, this could be achieved by increasing the number of ratchet teeth 29a formed inside the second gear wheel 29, so that the resilient pawls 27b of the first gear wheel 27 engage with the ratchet teeth 29a essentially immediately upon opening of the mouthpiece cover 7. Other forms of one-way clutch will be readily apparent to those of ordinary skill in the art.

Consequently, the dispensing mechanism of the inhaler 1 is driven via the first and second gear wheels 27, 29 when the mouthpiece cover 7 is opened, but not when the mouthpiece cover 7 is closed.

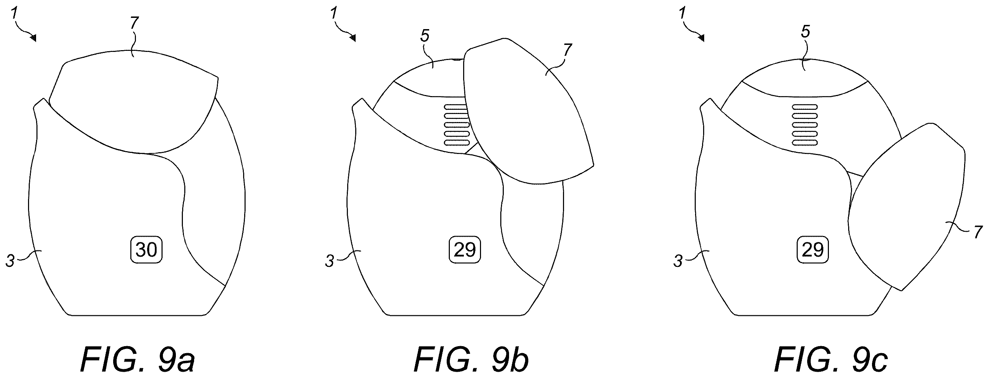

As mentioned hereinabove, the mouthpiece cover 7 of the second aspect of the invention is coupled to the first gear wheel 27 via a non-linear transmission means, and this non-linear transmission means will now be described with reference to Figs. 9a to 11.

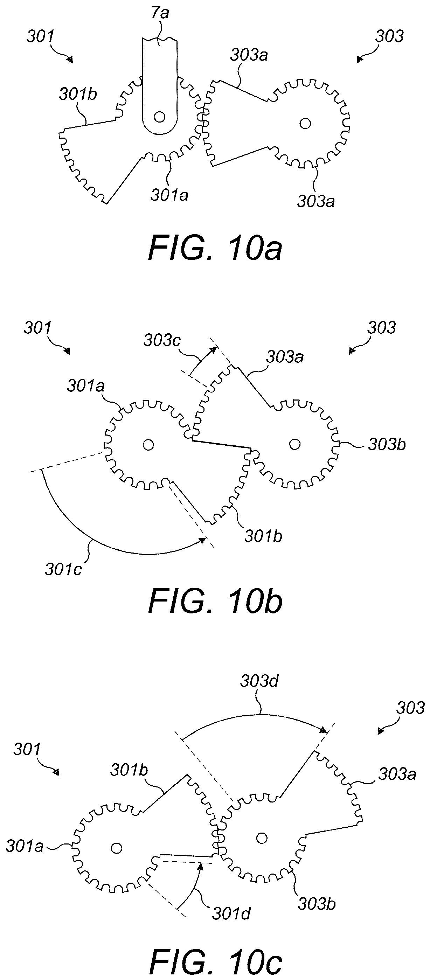

Figs 9a to 9c are schematic views showing the inhaler 1 with its mouthpiece cover 7 in first, second and third positions. Figs. 10a to 10c are schematic views showing the non-linear transmission means of the inhaler 1 when the mouthpiece cover 7 is in the first, second and third positions. It is noted that Figs. 10a to 10c show an opposite (back) side of the inhaler and/or its components to that shown in Figs. 9a to 9c (the front side).

Referring first to Figs. 9a and 10a, these drawings show the inhaler 1 and the non-linear transmission means that couples the mouthpiece cover 7 to the first gear wheel 27 when the mouthpiece cover 7 is in its first position, which is the normal position when the inhaler 1 is not in use. In this position, the mouthpiece 5 is completely covered by the mouthpiece cover 7 and the mouthpiece cover 7 is rotated to the maximum extent possible in the counter-clockwise direction (Fig. 9a).

With reference to Fig. 10a, it will be noted that the mouthpiece cover 7, in particular the hub 7a of the mouthpiece cover 7, is directly connected to an input gear wheel 301 such that the input gear wheel 301 rotates with the mouthpiece cover 7. The first gear wheel 27 is directly connected to an output gear wheel 303 so that the first gear wheel 27 rotates with the output gear wheel 303. The input and output gear wheels 301, 303 are in meshed engagement.

The input and output gear wheels 301, 303 have respective first segments 301a, 303a having first pitch circle diameters that define a first, relatively low gear ratio. The input and output gear wheels 301, 303 also have respective second segments 301b, 303b having second pitch circle diameters that define a second, relatively higher gear ratio. When the mouthpiece cover 7 is in the first position, the first segments 301a, 303a are in meshed engagement. It is noted that herein the term "gear ratio" is used to mean a ratio between the pitch circle diameters of gear wheels, so that this term does not have the same meaning as the term "movement ratio" also used herein.

Figs. 9b and 10b show the inhaler 1 and the non-linear transmission means after the mouthpiece cover 7 has been moved from the first position to the second position. It is noted that the hub 7a of the mouthpiece cover has been omitted from Fig. 10b (and Fig. 10c) for clarity reasons. In this position, the mouthpiece cover 7 has been rotated clockwise (or forwards) to part-uncover the mouthpiece 5, as shown in Fig. 9b. Movement from the first position to the second position may enclose an angle of from 20 to 160 degrees, and preferably from 30 to 90 degrees. In the embodiment illustrated, the angle enclosed is approximately 45 degrees.

With reference to Fig. 10b, it can be seen that movement of the mouthpiece cover 7 has been transmitted to the first gear wheel 27 through engagement of the first segments 301a, 303a of the input and output gear wheels 301, 303, with the first, relatively low gear ratio. In particular, rotation of the input gear wheel 301 through angle 301c has caused rotation of the output gear wheel 303 through angle 303c, which is smaller than angle 301c. This opening rotation of the output gear wheel 303 is transmitted to the first gear wheel 27, which in turn is transmitted to the second gear wheel 29 for driving the dispensing mechanism.

In summary, therefore, movement of the mouthpiece cover 7 from the first position to the second position has driven the dispensing mechanism of the inhaler 1 such that the next medicament-containing blisters of the medicament carriers 201a, 201b have been moved part way towards the dispensing position 19 of the inhaler 1 with a first, relatively low movement ratio.

Figs. 9c and 10c show the inhaler 1 and the non-linear transmission means after the mouthpiece cover 7 has been moved from the second position to the third position. In this position, the mouthpiece cover 7 has been rotated further clockwise (or forwards) to fully uncover the mouthpiece 5, as shown in Fig. 9c. Movement from the second position to the third position may enclose an angle of from 20 to 160 degrees, and preferably from 30 to 90 degrees. In the embodiment illustrated, the angle enclosed is approximately 45 degrees.

With reference to Fig. 10c, it can be seen that movement of the mouthpiece cover 7 has been transmitted to the first gear wheel 27 through engagement of the second segments 301b, 303b of the input and output gear wheels 301, 303, with the second, relatively higher gear ratio. In particular, rotation of the input gear wheel 301 through angle 301d has caused rotation of the output gear wheel 303 through angle 303d, which is greater than angle 301d. This further opening rotation of the output gear wheel 303 is transmitted to the first gear wheel 27, which in turn is transmitted to the second gear wheel 29 for further driving of the dispensing mechanism.

To further summarise, therefore, movement of the mouthpiece cover 7 from the second position to the third position has driven the dispensing mechanism such that the next medicament-containing blisters of the medicament carriers 201a, 201b have been further moved all the way to the dispensing position 19 of the inhaler 1, with a second, relatively higher movement ratio.

**It** is noted that, in the inhaler 1 of this aspect, the non-linear transmission means adds an additional gear wheel to the overall gear train, as compared to the inhaler 1 of the first aspect. This has the effect of changing the rotation direction of the first and second gear wheels 27, 29, so that suitable further alterations have to be made to ensure that the indexing wheels 15a, 15b, peeling spools 17a, 17b and coiling spools 23a, 23b rotate in the correct direction. This can be achieved by introducing an additional idler gear wheel (not shown) between the second gear wheel 29 and the rest of the dispensing mechanism to cause a further change in direction, or by reconfiguring the sequential order of the gear train.

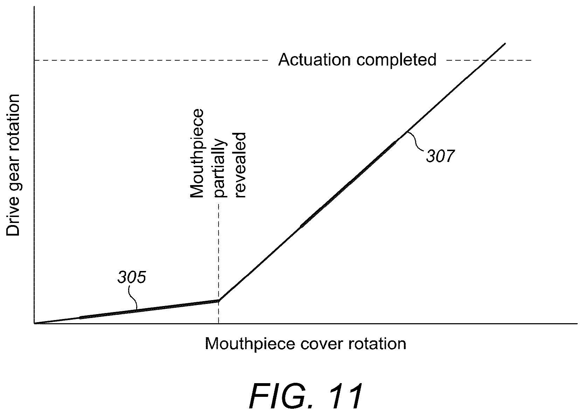

Fig. 11 is a graphical representation of operation of the non-linear transmission means shown in Figs. 10a to 10c. It can be seen that the dispensing mechanism is actuated in a non-linear manner, initially with a first, lower movement ratio 305, and then with a second, higher movement ratio 307.

The inhaler 1 of the second aspect provides an arrangement which allows for improved operation of the inhaler by providing a more adaptable dispensing mechanism. In particular, when the mouthpiece cover 7 is opened, the user does not experience a sudden resistance when the dispensing mechanism is engaged, but rather the increase in resistance is more gradual or progressive. This helps to protect the mouthpiece cover 7 and components of the dispensing mechanism from damage by avoiding excessive stress, and the resulting smoother operation helps to avoid vibration-induced movement of the powder medicament, which could otherwise result in poor dosing consistency and contamination of the internal components of the inhaler 1.

A further advantage of the inhaler 1 described above is that by initially driving the dispensing mechanism at a lower rate (i.e. movement ratio), it is more likely that any actuation caused by unintentional or accidental movement of the mouthpiece cover 7 between the first and second positions will be reversible. In particular, it is more likely that the unintentional or accidental movement will not have caused the next medicament-containing blisters to be peeled open.

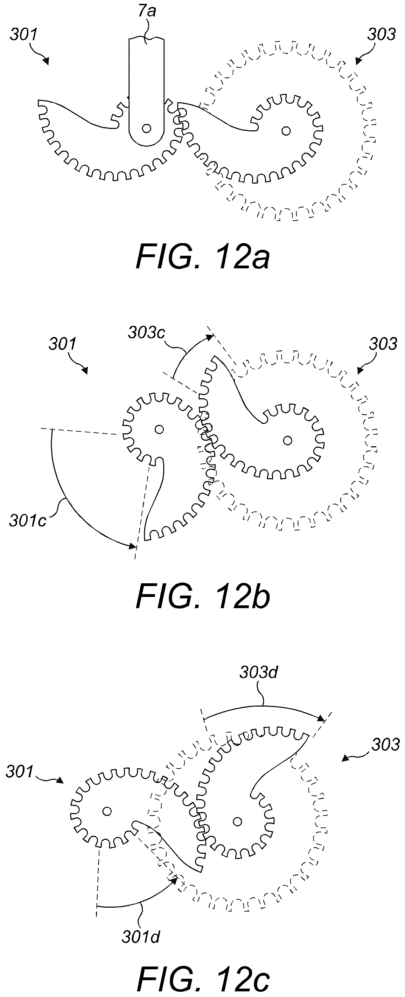

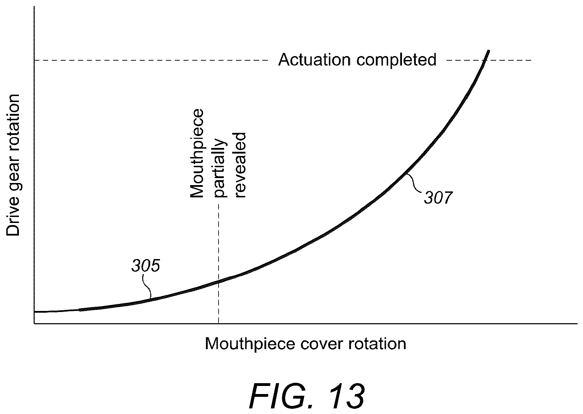

A number of modifications to the inhaler 1 of the second aspect are possible. Figs. 12a to 12c are schematic views showing a non-linear transmission means of a first alternative embodiment of the inhaler 1 when the mouthpiece cover is in the first, second and third positions (as shown in Figs. 9a to 9c). Fig. 13 is a graphical representation of operation of the non-linear transmission means shown in Figs. 12a to 12c.

The inhaler 1 of the first alternative embodiment is the same as that described above, except that the input gear wheel 301 and the output gear wheel 303 define a varying gear ratio that gradually increases, without any step changes, as the mouthpiece cover 7 is opened.

With reference to Fig. 12b, it can be seen that movement of the mouthpiece cover 7 has been transmitted to the first gear wheel 27 through engagement of the input and output gear wheels 301, 303, with a relatively low, but increasing, gear ratio. In particular, rotation of the input gear wheel 301 through angle 301c has caused rotation of the output gear wheel 303 through angle 303c, which is smaller than angle 301c.

With reference to Fig. 12c, it can be seen that further movement of the mouthpiece cover 7 has been transmitted to the first gear wheel 27 through engagement of the input and output gear wheels 301, 303, with a relatively higher, and still increasing gear ratio. In particular, rotation of the input gear wheel 301 through angle 301d has caused rotation of the output gear wheel 303 through angle 303d, which is greater than angle 301d.

With reference to Fig. 13, it can be seen that the dispensing mechanism according to this alternative embodiment is actuated in a non-linear manner, initially with a first, lower movement ratio 305, and then with a second, higher movement ratio 307.

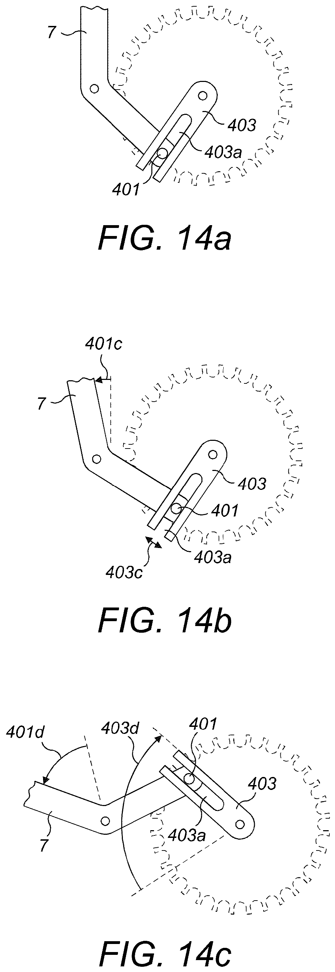

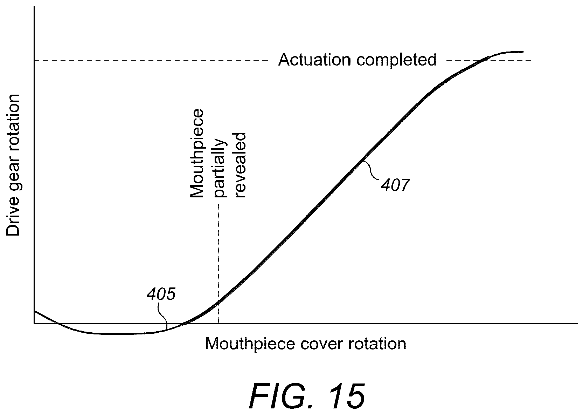

Figs. 14a to 14c are schematic views showing a non-linear transmission means of a second alternative embodiment of the inhaler 1 when the mouthpiece cover is in the first, second and third positions (as shown in Figs. 9a to 9c). Fig. 15 is a graphical representation of operation of the non-linear transmission means shown in Figs. 14a to 14c.

The inhaler 1 of the second alternative embodiment is the same as that described above, except that the input gear wheel 301 is replaced with a pin 401 eccentrically mounted to the mouthpiece cover 7 (shown in highly schematic form in Figs. 14a to 14c) and the output gear wheel 303 is replaced with a gear wheel 403 (also shown in highly schematic form in Figs. 14a to 14c) having a radially extending slot 403a for receiving the pin 401. During opening of the mouthpiece cover 7, the pin 401 is arranged to engage the slot 403a to thereby rotate the gear wheel 403. The interaction between the pin 401 and gear wheel 403 defines a gear ratio that varies, without any step changes, as the mouthpiece cover 7 is opened.

With reference to Fig. 14b, it can be seen that movement of the mouthpiece cover 7 has been transmitted to the first gear wheel 27 through engagement of the pin 401 and slot 403a, with a relatively low, gear ratio and in a direction that reverses (see also Fig. 15). In particular, rotation of the pin 401 (and mouthpiece cover 7) through angle 401c has caused back-and-forth rotation of the gear wheel 403 through angle 403c.

With reference to Fig. 14c, it can be seen that further movement of the mouthpiece cover 7 has been transmitted to the first gear wheel 27 through engagement of the pin 401 and slot 403a, with a higher gear ratio. In particular, rotation of the pin 401 through angle 401d has caused rotation of the gear wheel 403 through angle 403d.

With reference to Fig. 15, it can be seen that the dispensing mechanism according to this alternative embodiment is actuated in a non-linear manner, initially with a first, lower movement ratio 405, and then with a second, higher movement ratio 407.

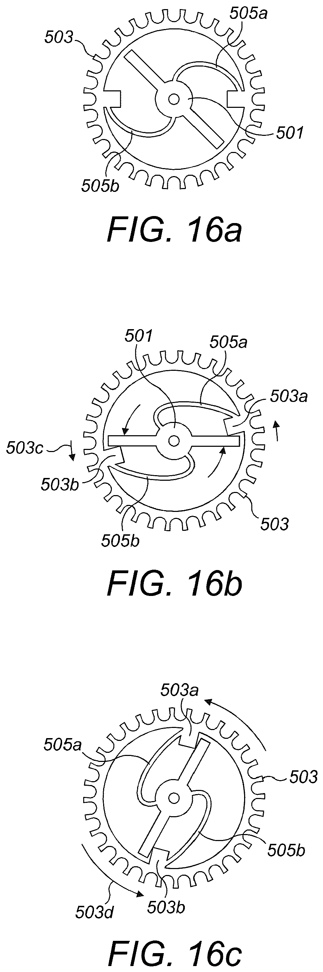

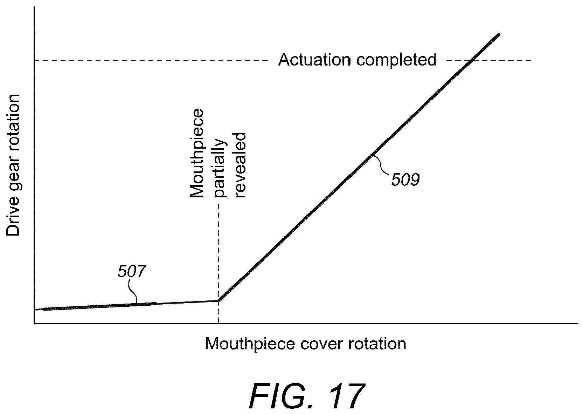

Figs. 16a to 16c are schematic views showing a non-linear transmission means of a third alternative embodiment of the inhaler 1 when the mouthpiece cover is in the first, second and third positions (as shown in Figs. 9a to 9c). Fig. 17 is a graphical representation of operation of the non-linear transmission means shown in Figs. 16a to 16c.

The inhaler 1 of the third alternative embodiment is the same as that described above, except that the input gear wheel 301 is replaced with a hub 501 mounted to the mouthpiece cover 7 and the output gear wheel 303 is replaced with an annular gear wheel 503. The hub 501 and the gear wheel 503 are resiliently coupled by spring elements 505a, 505b, such that movement of the mouthpiece cover 7 from the first position to the second position causes torsional deformation of the resilient coupling 505a, 505b along with some transmission of rotation from the hub 501 to the gear wheel 503 (see arrow 503c). Further movement of the mouthpiece cover 7 from the second position to the third position causes no further torsional deformation of the resilient coupling 505a, 505b owing to direct engagement between the hub 501 and a pair of internal teeth 503a, 503b of the gear wheel 503. This movement of the mouthpiece cover 7 instead results in a greater transmission of rotation from the hub 501 to the gear wheel 503 (see arrow 503d).

With reference to Fig. 17, it can be seen that the dispensing mechanism according to this alternative embodiment is actuated in a non-linear manner, initially with a first, lower movement ratio 507, and then with a second, higher movement ratio 509.

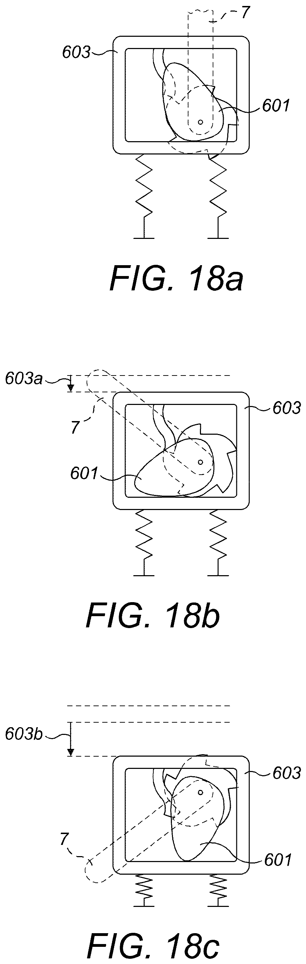

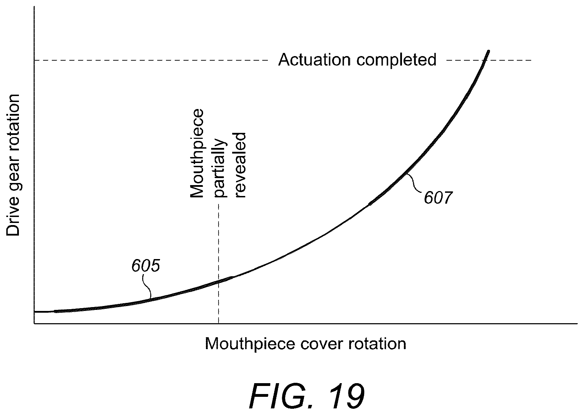

Figs. 18a to 18c are schematic views showing another non-linear transmission means that may be used in the inhaler 1 of the first or second aspect of the invention, and Fig. 19 is an exemplary graphical representation of its operation.

The non-linear transmission means shown in Figs. 18a to 18c comprises a cam 601 which rotates with the mouthpiece cover 7 and a resiliently-biased follower 603 which is arranged for reciprocating movement in the direction of arrows 603a, 603b. The follower carriers a pawl 603b which engages a gear wheel for driving the dispensing mechanism.

Fig. 19 is an exemplary graphical representation of the non-linear transmission's operation. **It** can be seen that the dispensing mechanism is actuated in a non-linear manner, initially with a first, lower movement ratio 605, and then with a second, higher movement ratio 607, in line with the principles of the second aspect of the invention. However, it will be appreciated that the profile of the cam 601 could be modified to change the transmission properties. This includes modifying the cam profile to include a segment having a constant diameter so that movement of the mouthpiece cover 7 from the first position to the second position does not result in any movement of the medicament dose, in line with the principles of the first aspect of the invention.

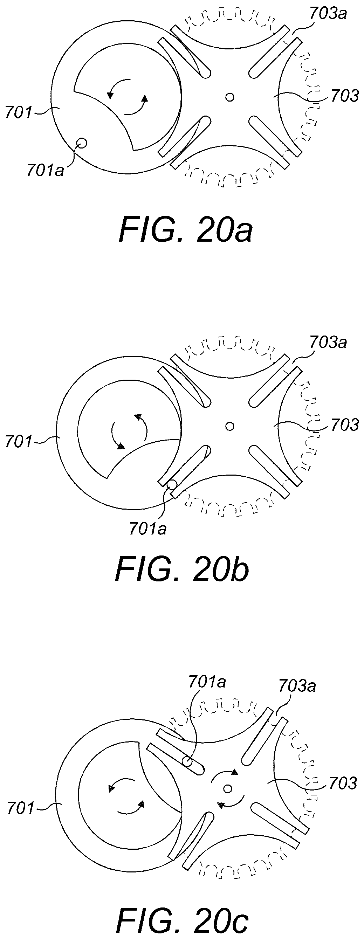

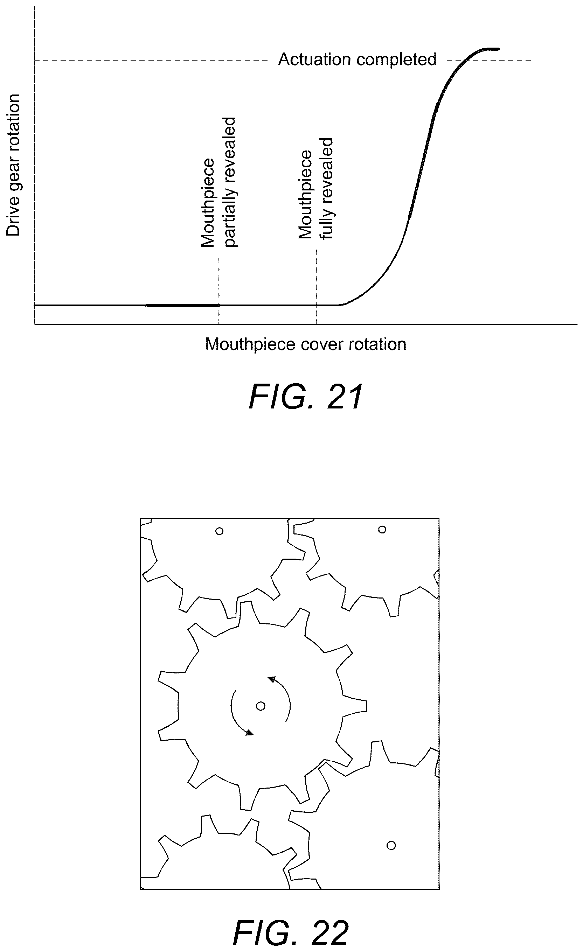

Figs. 20a to 20c are schematic views showing another non-linear transmission means that may be used in the inhaler 1 according to the first or second embodiment, and Fig. 21 is an exemplary graphical representation of its operation.

The non-linear transmission means shown in Figs. 20a to 20c is a Geneva drive. The Geneva drive comprises a first gear wheel 701 carrying an eccentrically-mounted pin 701a, which is driven by the mouthpiece cover 7, and a second gear wheel 703 provided with a plurality of slots 703a, which drives the dispensing mechanism. The coupling between the mouthpiece cover 7 and the first gear wheel 701 is typically via a one-way clutch (not shown), so that the first gear wheel 701 is only driven by opening of the mouthpiece cover 7, not by closing it. When the mouthpiece cover 7 is opened, the pin 701a of the first gear wheel 701 engages a slot 703 of the second gear wheel 703 to thereby transmit the rotational motion.

Further information on the Geneva drive can be found in, for example, Theory of Machines and Mechanisms, Shigley et al., second edition, 1995, McGraw-Hill, Inc. (pages 373 to 376).

Fig. 21 is an exemplary graphical representation of the non-linear transmission's operation. It can be seen that the dispensing mechanism is actuated in a non-linear manner, in line with the principles of the second aspect of the invention. It can also be seen that movement of the mouthpiece cover 7 from the first position to the second position does not result in any movement of the medicament dose, in line with the principles of the first aspect of the invention.

Fig. 22 is a schematic view showing another transmission means that may be used in the inhaler 1 according to the first embodiment. The transmission means comprises a gear train in which the gear teeth are designed to exhibit a large amount of backlash. This can be achieved, for example, by spacing the gear teeth further apart than normal and configuring the gear wheels to have relatively large diameters (so as to reduce curvature and maintain suitable engagement). The effect of these adaptations is to delay the transmission of rotational movement so that the dispensing mechanism is not driven when the mouthpiece cover 7 is moved from its first position to its second position, in line with the principles of the first aspect of the invention.

In any of the embodiments described above, the medicament carriers 201a, 201b may be provided in a removable cartridge received by the housing 3 of the inhaler 1. The medicament carriers may comprise respective different medicaments for simultaneous inhalation by the user. For example, the different medicaments for simultaneous inhalation may comprise: LABA or SABA in a first medicament carrier and an ICS in a second medicament carrier; or LABA or SABA in a first medicament carrier and LAMA or SAMA in a second medicament carrier; or LABA or SABA and LAMA or SAMA in a first medicament carrier and ICS in a second medicament carrier, or budesonide in a first medicament carrier and formoterol in a second medicament carrier; or beclomethasone in a first medicament carrier and formoterol in a second medicament carrier; or fluticasone in a first medicament carrier and salmeterol in a second medicament carrier, or fluticasone in a first medicament carrier and albuterol in a second medicament carrier, or fluticasone in a first medicament carrier and vilanterol in a second medicament carrier, or umeclidinium in a first medicament carrier and vilanterol in a second medicament carrier, or two selected from umeclidinium, fluticasone and vilanterol in a first medicament carrier, the remaining medicament from umeclidinium, fluticasone and vilanterol in a second medicament carrier.

In particular, the different medicaments for simultaneous inhalation may comprise: fluticasone furoate in a first medicament carrier and vilanterol trifenatate in a second medicament carrier, or umeclidinium bromide in a first medicament carrier and vilanterol trifenatate in a second medicament carrier, or two selected from umeclidinium bromide, fluticasone furoate and vilanterol trifenatate in a first medicament carrier and the remaining medicament from umeclidinium bromide, fluticasone furoate and vilanterol trifenatate in a second medicament carrier.

Other medicaments that may be used include for example any one of long acting beta antagonists (LABA) , short acting beta antagonists (SABA), corticosteroids (ICS), long acting muscarinic antagonists (LAMA), short acting muscarinic antagonists (SAMA), or any other drug that can be administered via inhalation including combination thereof. Examples for such drugs include but are not limited to budesonide, formoterol, beclomethasone, fluticasone, salmeterol, albuterol, salbutamol, indacaterol, Tiotropium, ipratropium, glycorpyrronium , umeclidinium and combination thereof.

Although the inhalers 1 described above comprise two medicament carriers 201a, 201b, alternative embodiments of the invention may comprise a single medicament carrier, in which case only one half of the dispensing mechanism would be need to be provided. In this case, each of the spaced-apart medicament doses comprises budesonide, formoterol, beclomethasone, fluticasone, salmeterol, albuterol or umeclidinium.

Alternatively, the single medicament carrier containing embodiment of the invention may be configured for simultaneous inhalation of different medicaments by configuring the dispensing mechanism to open two blisters at a time, and providing different blisters of the medicament carrier with different medicaments. For example, the different medicaments for simultaneous inhalation may comprise: budesonide in a first blister and formoterol in a second blister; or beclomethasone in a first blister and formoterol in a second blister; or fluticasone in a first blister and salmeterol in a second blister, or fluticasone in a first blister and albuterol in a second blister, or fluticasone in a first blister and vilanterol in a second blister, or umeclidinium in a first blister and vilanterol in a second blister, or two selected from umeclidinium, fluticasone and vilanterol in a first blister, the remaining medicament from umeclidinium, fluticasone and vilanterol in a second blister.

In particular, the different medicaments for simultaneous inhalation comprise: fluticasone furoate in a first blister and vilanterol trifenatate in a second blister, or umeclidinium bromide in a first blister and vilanterol trifenatate in a second blister, or two selected from umeclidinium bromide, fluticasone furoate and vilanterol trifenatate in a first blister and the remaining medicament from umeclidinium bromide, fluticasone furoate and vilanterol trifenatate in a second blister.

Several non-limiting embodiments of the invention have been described hereinabove with reference to the accompanying drawings. Various changes may be made to these embodiments without departing from the scope of the invention, which is defined by the claims.

For example, the inhalers described hereinabove are for use with medicament carriers in the form of blister packs that are peeled open. However, the inhalers may be for use with blister packs that are opened by piercing or bursting, or with completely different types of medicament carrier, provided they have spaced-apart doses of medicament. The medicament carriers described above are in the form of elongate strips. In other embodiments, the medicament carriers may be in the form of discs having the medicament doses arranged about the circumference.

The following embodiments, which were claimed in the parent application, also form a part of the present disclosure:
1. A dry powder inhaler for delivering medicament from at least one medicament carrier having a plurality of spaced-apart medicament doses, the dry powder inhaler comprising:
   a housing having regions for accommodating unused and used portions of the medicament carrier;
   a mouthpiece component joined to the housing and through which a user is able to inhale, the mouthpiece component including surfaces for contacting the user's lips;
   a dispensing mechanism arranged in the housing for moving a next medicament dose of the medicament carrier to a dispensing position; and
   a mouthpiece cover rotatably connected to the housing for sequential movement about a rotation axis from a first position in which the mouthpiece component is completely covered to a second position, and from the second position to a third position in which the mouthpiece component is completely uncovered, the second position being between the first and third positions,
   wherein the mouthpiece cover is coupled to the dispensing mechanism such that movement of the mouthpiece cover drives the dispensing mechanism,
   wherein the coupling is arranged such that movement of the mouthpiece cover from the first position to the second position does not cause the next medicament dose of the medicament carrier to move, and movement of the mouthpiece cover from the second position to the third position does cause the next medicament dose of the medicament carrier to be moved to the dispensing position, with the next medicament dose starting to move to the dispensing position when the mouthpiece cover moves through the second position, wherein movement from the first position to the second position encloses an angle about the rotation axis of at least 5 degrees, and the mouthpiece component is completely covered when the mouthpiece cover is in the second position.
2. The dry powder inhaler according to embodiment 1, wherein movement of the mouthpiece cover from the first position to the second position encloses an angle about the rotation axis of from 5 to 30 degrees, optionally from 5 to 15 degrees, further optionally from 8 to 15 degrees.
3. The dry powder inhaler according to embodiment 1 or 2, wherein movement of the mouthpiece cover from the second position to a position at which the mouthpiece component starts to be exposed encloses an angle of at least 5 degrees, optionally from 5 to 30 degrees, further optionally from 5 to 15 degrees, further optionally from 8 to 15 degrees.
4. The dry powder inhaler according to any preceding embodiment, wherein the first position of the mouthpiece cover is a fully closed position from which the mouthpiece cover is only able to rotate in one direction.
5. The dry powder inhaler according to any preceding embodiment, wherein movement of the mouthpiece cover causes movement of at least one resilient drive pawl, and wherein the at least one resilient drive pawl selectively engages a toothed drive gear of the dispensing mechanism for driving the dispensing mechanism to move the next medicament dose of the medicament carrier to the dispensing position.
6. The dry powder inhaler according to embodiment 5, wherein, on movement of the mouthpiece cover from the first position to the second position, the at least one resilient drive pawl is not engaged with any teeth of the drive gear, and, on movement of the mouthpiece cover from the second position to the third position, the at least one resilient drive pawl is engaged with at least one tooth of the drive gear.
7. The dry powder inhaler according to embodiment 5 or 6, wherein the at least one resilient drive pawl is provided about a first gear wheel which is rotatably driven by the mouthpiece cover, and the drive gear is provided as ratchet teeth about a second gear wheel arranged to rotate coaxially with the first gear wheel, optionally wherein the at least one resilient drive pawl is provided about the outside of the first gear wheel and the drive gear is provided as an internal gear about the second gear wheel.
8. The dry powder inhaler according to embodiment 7, wherein the first gear wheel is arranged to rotate forwards and backwards when the mouthpiece cover is opened and closed, respectively, and wherein the second gear wheel is arranged to rotate forwards when driven forwards by the first gear wheel and not to rotate backwards, optionally wherein the second gear wheel is provided with a ratchet and pawl arrangement for preventing reverse rotation.
9. The dry powder inhaler according to embodiment 7 or 8, wherein:
   the first gear wheel is provided with four resilient pawls at 90 degree intervals and the second gear wheel is provided with four teeth at 90 degree intervals, and movement of the mouthpiece cover from the second position to the third position encloses an angle about the rotation axis of 90 degrees; or
   the first gear wheel is provided with five resilient pawls at 72 degree intervals and the second gear wheel is provided with five teeth at 72 degree intervals, and movement of the mouthpiece cover from the second position to the third position encloses an angle about the rotation axis of 72 degrees.
10. A dry powder inhaler for delivering medicament from at least one medicament carrier having a plurality of spaced-apart medicament doses, the dry powder inhaler comprising:
   a housing having regions for accommodating unused and used portions of the medicament carrier;
   a mouthpiece component joined to the housing and through which a user is able to inhale, the mouthpiece component including surfaces for contacting the user's lips;
   a dispensing mechanism arranged in the housing for moving a next medicament dose of the medicament carrier to a dispensing position; and
   a mouthpiece cover rotatably connected to the housing for sequential movement about a rotation axis from a first position in which the mouthpiece component is at least partly covered to a second position, and from the second position to a third position in which the mouthpiece component is completely uncovered, the second position being between the first and third positions,
   wherein the mouthpiece cover is coupled to the dispensing mechanism such that movement of the mouthpiece cover drives the dispensing mechanism, with movement of the mouthpiece cover from the first position to the third position causing the next medicament dose of the medicament carrier to be moved to the dispensing position,
   wherein the coupling of the mouthpiece cover to the dispensing mechanism is arranged to be non-linear, such that movement of the mouthpiece cover from the first position to the second position causes the next medicament dose to be continuously moved to an intermediate position with a first movement ratio, and movement of the mouthpiece cover from the second position to the third position causes the next medicament dose to be continuously moved from the intermediate position to the dispensing position with a second movement ratio which is different to the first movement ratio.
11. The dry powder inhaler according to embodiment 10, wherein the mouthpiece component is completely covered when the mouthpiece cover is in the first position, and wherein the mouthpiece component is partly covered when the mouthpiece cover is in the second position.
12. The dry powder inhaler according to embodiment 10, wherein the mouthpiece component is completely covered when the mouthpiece cover is in the first position, and wherein the mouthpiece component is completely covered when the mouthpiece cover is in the second position.
13. The dry powder inhaler according to any of embodiments 10 to 12, wherein the first position of the mouthpiece cover is a fully closed position from which the mouthpiece cover is only able to rotate in one direction.
14. The dry powder inhaler according to embodiment 10, wherein the mouthpiece component is partly covered when the mouthpiece cover is in the first position, and the mouthpiece component is also partly covered when the mouthpiece cover is in the second position.
15. The dry powder inhaler according to any of embodiments 10 to 14, wherein movement from the first position to the second position encloses an angle about the rotation axis of from 20 to 160 degrees, optionally from 30 to 90 degrees, and movement from the second position to the third position encloses an angle about the rotation axis of from 20 to 160 degrees, optionally from 30 to 90 degrees.
16. The dry powder inhaler according to any of embodiments 10 to 15, wherein the second movement ratio is greater than the first movement ratio.
17. The dry powder inhaler according to any of embodiments 10 to 16, wherein the mouthpiece cover drives a first gear wheel and the dispensing mechanism is driven by a second gear wheel, the first and second gear wheels forming a gear train that is in meshed engagement at least during opening of the mouthpiece cover.
18. The dry powder inhaler of embodiment 17, and wherein the first and second gear wheels have respective first segments having first pitch circle diameters that define a first gear ratio corresponding to the first movement ratio, and the first and second gear wheels have respective second segments having second pitch circle diameters that define a second gear ratio corresponding to the second movement ratio.
19. The dry powder inhaler according to embodiment 17, wherein the first and second gear wheels have respective segments with pitch circle diameters that vary, without step changes, such that a gear ratio between the first and second gear wheels varies, without step changes.
20. The dry powder inhaler according to embodiment 17, wherein the first and second gear wheels form a Geneva drive.
21. The dry powder inhaler according to embodiment 16, wherein the first gear wheel carries a pin of the Geneva drive and the second gear wheel is provided with a plurality of slots of the Geneva drive for engagement by the pin.
22 The dry powder inhaler according to any of embodiments 10 to 16, wherein the mouthpiece cover drives a rotatable cam and the dispensing mechanism is driven by a follower, wherein the cam engages the follower, such that movement of the mouthpiece cover from the first position to the third position causes the next medicament dose to be moved to the dispensing position.
23. The dry powder inhaler according to any of embodiments 10 to 16, wherein the mouthpiece cover includes an eccentrically mounted pin, and dispensing mechanism is driven by a gear wheel having a radially-extending slot for receiving the pin, and wherein, during opening of the mouthpiece cover, the pin is arranged to engage the slot to thereby rotate the gear wheel, such that movement of the mouthpiece cover from the first position to the third position causes the next medicament dose to be moved to the dispensing position.
24. The dry powder inhaler according to embodiment 16, wherein the dispensing mechanism is driven by a gear wheel, a hub associated with the gear wheel being rotationally driven by the opening of the mouthpiece cover, and wherein the hub and the gear wheel resiliently coupled, such that movement of the mouthpiece cover from the first position to the second position causes torsional deformation of the resilient coupling along with movement of the next medicament dose with the first movement ratio, and movement of the mouthpiece cover from the second position to the third position causes a lesser amount of torsional deformation of the resilient coupling along with movement of the next medicament dose with the second movement ratio.
25. The dry powder inhaler according to embodiment 24, wherein, during movement of the mouthpiece cover from the second position to the third position, the hub directly engages the gear wheel so that there is no further torsional deformation of the resilient coupling.
26. The dry powder inhaler according to any of embodiments 19 to 25, wherein movement of the mouthpiece cover from the first position to the third position causes the dispensing mechanism to be driven with a gear ratio that varies, without any step changes in the gear ratio.
27. The dry powder inhaler according to any of embodiments 19 to 26, wherein the gear ratio increases from the first position to the third position of the mouthpiece cover.
28. The dry powder inhaler according to any of embodiments 17 to 27, wherein the dispensing mechanism comprises an anti-reverse rotation mechanism, such that opening movement of the mouthpiece cover from the first position to the third position causes the next medicament dose to be moved, and closing movement of the mouthpiece cover from the third position to the first position does not causes the next medicament dose to be moved, optionally wherein the anti-reverse rotation mechanism comprises a ratchet and pawl arrangement.
29. The dry powder inhaler according to any preceding embodiment, further comprising a manifold arranged in the housing, the manifold defining at least part of an air flow path extending from an air inlet to the mouthpiece component, the manifold also defining the dispensing position for the next medicament dose of the medicament carrier.
30. The dry powder inhaler according to embodiment 29, wherein the dispensing mechanism is also arranged for placing the next medicament dose at the dispensing position in fluid communication with the air flow path, ready for inhalation by the user, optionally by selectively peeling or piercing a portion of the medicament carrier.
31. The dry powder inhaler according to any preceding embodiment, wherein the medicament carrier comprises an elongate blister strip having a base layer defining spaced-apart blister openings containing medicament doses, and a cover layer adhesively bonded to the base layer to close the blisters, and wherein the cover layer is arranged to be peeled from the base layer.
32. The dry powder inhaler according to embodiment 31, wherein the dispensing mechanism comprises:
   an indexing mechanism for moving a blister of the elongate blister strip containing the next medicament dose to the dispensing position; and
   a peeling mechanism for peeling the cover layer from the blister for placing the medicament dose in fluid communication with the air flow path ready for inhalation by the user.
33. The dry powder inhaler according to any preceding embodiment, further comprising the medicament carrier, optionally wherein the dry powder inhaler comprises a single medicament carrier, and further optionally wherein the medicament carrier is arranged in a replaceable cartridge received by the housing.
34. The dry powder inhaler of embodiment 33, wherein each of the spaced-apart medicament doses comprises budesonide, formoterol, beclomethasone, fluticasone, salmeterol, albuterol, salbutamol, indacaterol, tiotropium, ipratropium, glycorpyrronium or umeclidinium, vilanterol, or any combination thereof.
35. The dry powder inhaler according to embodiment 32, wherein the indexing mechanism is arranged to move at least two blisters of the elongate blister strip containing the next medicament doses to the dispensing position, and the peeling mechanism is arranged to peel the cover layer from the at least two blisters for placing the at least two medicament doses in fluid communication with the air flow path ready for inhalation by the user, such that the dry powder inhaler can be used for simultaneous inhalation of different medicaments from different blisters.
36. The dry powder inhaler according to embodiment 35, further comprising the medicament carrier, optionally wherein the dry powder inhaler comprises a single medicament carrier, and further optionally wherein the medicament carrier is arranged in a replaceable cartridge received by the housing.
37. The dry powder inhaler according to embodiment 36, wherein the different medicaments for simultaneous inhalation comprise:
   LABA or SABA in a first blister and an ICS in a second blister; or
   LABA or SABA in a first blister and LAMA or SAMA in a second blister; or
   LABA or SABA and LAMA or SAMA in a first blister and ICS in a second blister, or
   budesonide in a first blister and formoterol in a second blister; or
   beclomethasone in a first blister and formoterol in a second blister; or
   fluticasone in a first blister and salmeterol in a second blister, or
   fluticasone in a first blister and albuterol in a second blister, or
   fluticasone in a first blister and vilanterol in a second blister, or
   umeclidinium in a first blister and vilanterol in a second blister, or
   two selected from umeclidinium, fluticasone and vilanterol in a first blister, the remaining medicament from umeclidinium, fluticasone and vilanterol in a second blister.
38. The dry powder inhaler according to embodiment 36 or 37, wherein the different medicaments for simultaneous inhalation comprise:
   fluticasone furoate in a first blister and vilanterol trifenatate in a second blister, or
   umeclidinium bromide in a first blister and vilanterol trifenatate in a second blister, or
   two selected from umeclidinium bromide, fluticasone furoate and vilanterol trifenatate in a first blister and the remaining medicament from umeclidinium bromide, fluticasone furoate and vilanterol trifenatate in a second blister.
39. The dry powder inhaler according to any of embodiments 1 to 30, for delivering medicament from a plurality of medicament carriers each having a plurality of spaced-apart medicament doses.
40. The dry powder inhaler according to embodiment 39, wherein each of the medicament carriers comprises an elongate blister strip having a base layer defining spaced-apart blister openings containing medicament doses, and a cover layer adhesively bonded to the base layer to close the blisters, and wherein the cover layer is arranged to be peeled from the base layer.
41. The dry powder inhaler according to embodiment 40, wherein the dispensing mechanism comprises:
   an indexing mechanism for moving a blister of each of the elongate blister strips containing the next medicament to the dispensing position; and
   a peeling mechanism for peeling the cover layer from each of the blister strips for placing the medicament doses in fluid communication with the air flow path ready for simultaneous inhalation by the user.
42. The dry powder inhaler according to any of embodiments 39 to 41, further comprising the medicament carriers, optionally wherein the dry powder inhaler comprises exactly two medicament carriers, and further optionally wherein the medicament carriers are arranged in at least one replaceable cartridge received by the housing.
43. The dry powder inhaler of embodiment 42, wherein the medicament carriers comprise respective different medicament for simultaneous inhalation.
44. The dry powder inhaler according to embodiment 43, wherein the different medicaments for simultaneous inhalation comprise:
   LABA or SABA in a first medicament carrier and an ICS in a second medicament carrier; or
   LABA or SABA in a first medicament carrier and LAMA or SAMA in a second medicament carrier; or
   LABA or SABA and LAMA or SAMA in a first medicament carrier and ICS in a second medicament carrier, or
   budesonide in a first medicament carrier and formoterol in a second medicament carrier; or
   beclomethasone in a first medicament carrier and formoterol in a second medicament carrier; or
   fluticasone in a first medicament carrier and salmeterol in a second medicament carrier, or
   fluticasone in a first medicament carrier and albuterol in a second medicament carrier, or
   fluticasone in a first medicament carrier and vilanterol in a second medicament carrier, or
   umeclidinium in a first medicament carrier and vilanterol in a second medicament carrier, or
   two selected from umeclidinium, fluticasone and vilanterol in a first medicament carrier, the remaining medicament from umeclidinium, fluticasone and vilanterol in a second medicament carrier.
45. The dry powder inhaler according to embodiment 43 or 44, wherein the different medicaments for simultaneous inhalation comprise:
   fluticasone furoate in a first medicament carrier and vilanterol trifenatate in a second medicament carrier, or
   umeclidinium bromide in a first medicament carrier and vilanterol trifenatate in a second medicament carrier, or
   two selected from umeclidinium bromide, fluticasone furoate and vilanterol trifenatate in a first medicament carrier and the remaining medicament from umeclidinium bromide, fluticasone furoate and vilanterol trifenatate in a second medicament carrier.

## Claims

1. A dry powder inhaler for delivering medicament from at least one medicament carrier (201) having a plurality of spaced-apart medicament doses, the dry powder inhaler comprising:
a housing (3) having regions for accommodating unused and used portions (13a, 13b) of the medicament carrier (201);
a mouthpiece component (5) joined to the housing and through which a user is able to inhale, the mouthpiece component (5) including surfaces for contacting the user's lips;
a dispensing mechanism arranged in the housing for moving a next medicament dose of the medicament carrier (201) to a dispensing position; and
a mouthpiece cover (7) rotatably connected to the housing (3) for sequential movement about a rotation axis from a first position in which the mouthpiece component (5) is completely covered to a second position, and from the second position to a third position in which the mouthpiece component (5) is completely uncovered, the second position being between the first and third positions,
wherein the mouthpiece cover (7) is coupled to the dispensing mechanism such that movement of the mouthpiece cover (7) drives the dispensing mechanism,
wherein the coupling is arranged such that movement of the mouthpiece cover (7) from the first position to the second position does not cause the next medicament dose of the medicament carrier (201) to move, and movement of the mouthpiece cover (7) from the second position to the third position does cause the next medicament dose of the medicament carrier (201) to be moved to the dispensing position, with the next medicament dose starting to move to the dispensing position when the mouthpiece cover (7) moves through the second position, wherein movement from the first position to the second position encloses an angle about the rotation axis of at least 5 degrees, and the mouthpiece component (5) is completely covered when the mouthpiece cover (7) is in the second position.

2. The dry powder inhaler according to claim 1, wherein movement of the mouthpiece cover (7) from the first position to the second position encloses an angle about the rotation axis of from 5 to 30 degrees, optionally from 5 to 15 degrees, further optionally from 8 to 15 degrees; and/or wherein movement of the mouthpiece cover (7) from the second position to a position at which the mouthpiece component (5) starts to be exposed encloses an angle of at least 5 degrees, optionally from 5 to 30 degrees, further optionally from 5 to 15 degrees, further optionally from 8 to 15 degrees.

3. The dry powder inhaler according to any preceding claim, wherein the first position of the mouthpiece cover (7) is a fully closed position from which the mouthpiece cover (7) is only able to rotate in one direction.

4. The dry powder inhaler according to any preceding claim, wherein movement of the mouthpiece cover (7) causes movement of at least one resilient drive pawl, and wherein the at least one resilient drive pawl selectively engages a toothed drive gear of the dispensing mechanism for driving the dispensing mechanism to move the next medicament dose of the medicament carrier (201) to the dispensing position, optionally wherein, on movement of the mouthpiece cover (7) from the first position to the second position, the at least one resilient drive pawl is not engaged with any teeth of the drive gear, and, on movement of the mouthpiece cover from the second position to the third position, the at least one resilient drive pawl is engaged with at least one tooth of the drive gear.

5. The dry powder inhaler according to claim 4, wherein the at least one resilient drive pawl is provided about a first gear wheel (27) which is rotatably driven by the mouthpiece cover (7), and the drive gear is provided as ratchet teeth about a second gear wheel (29) arranged to rotate coaxially with the first gear wheel (27), optionally wherein the at least one resilient drive pawl is provided about the outside of the first gear wheel (27) and the drive gear is provided as an internal gear about the second gear wheel (29), further optionally wherein the first gear wheel (27) is arranged to rotate forwards and backwards when the mouthpiece cover (7) is opened and closed, respectively, and wherein the second gear wheel (29) is arranged to rotate forwards when driven forwards by the first gear wheel (27) and not to rotate backwards, optionally wherein the second gear wheel (29) is provided with a ratchet and pawl arrangement for preventing reverse rotation.

6. The dry powder inhaler according to claim 5, wherein:
the first gear wheel (27) is provided with four resilient pawls at 90 degree intervals and the second gear wheel (29) is provided with four teeth at 90 degree intervals, and movement of the mouthpiece cover from the second position to the third position encloses an angle about the rotation axis of 90 degrees; or
the first gear wheel (27) is provided with five resilient pawls at 72 degree intervals and the second gear wheel (29) is provided with five teeth at 72 degree intervals, and movement of the mouthpiece cover (7) from the second position to the third position encloses an angle about the rotation axis of 72 degrees.

7. A dry powder inhaler for delivering medicament from at least one medicament carrier (201) having a plurality of spaced-apart medicament doses, the dry powder inhaler comprising:
a housing (3) having regions for accommodating unused and used portions (13a, 13b) of the medicament carrier (201);
a mouthpiece component (5) joined to the housing and through which a user is able to inhale, the mouthpiece component (5) including surfaces for contacting the user's lips;
a dispensing mechanism arranged in the housing for moving a next medicament dose of the medicament carrier (201) to a dispensing position; and
a mouthpiece cover (7) rotatably connected to the housing (3) for sequential movement about a rotation axis from a first position in which the mouthpiece component (5) is at least partly covered to a second position, and from the second position to a third position in which the mouthpiece component (5) is completely uncovered, the second position being between the first and third positions,
wherein the mouthpiece cover (7) is coupled to the dispensing mechanism such that movement of the mouthpiece cover (7) drives the dispensing mechanism, with movement of the mouthpiece cover from the first position to the third position causing the next medicament dose of the medicament carrier to be moved to the dispensing position,
wherein the coupling of the mouthpiece cover (7) to the dispensing mechanism is arranged to be non-linear, such that movement of the mouthpiece cover (7) from the first position to the second position causes the next medicament dose to be continuously moved to an intermediate position with a first movement ratio, and movement of the mouthpiece cover (7) from the second position to the third position causes the next medicament dose to be continuously moved from the intermediate position to the dispensing position with a second movement ratio which is different to the first movement ratio.

8. The dry powder inhaler according to claim 7, wherein the first position of the mouthpiece cover is a fully closed position from which the mouthpiece cover is only able to rotate in one direction,
optionally wherein movement from the first position to the second position encloses an angle about the rotation axis of from 20 to 160 degrees, optionally from 30 to 90 degrees, and movement from the second position to the third position encloses an angle about the rotation axis of from 20 to 160 degrees, optionally from 30 to 90 degrees.

9. The dry powder inhaler according to claim 7 or 8, wherein the second movement ratio is greater than the first movement ratio.

10. The dry powder inhaler according to any of claims 7 to 9, wherein movement of the mouthpiece cover from the first position to the third position causes the dispensing mechanism to be driven with a gear ratio that varies, without any step changes in the gear ratio,
optionally wherein the gear ratio increases from the first position to the third position of the mouthpiece cover.

11. The dry powder inhaler according to any preceding claim, for delivering medicament from a plurality of medicament carriers (201) each having a plurality of spaced-apart medicament doses.

12. The dry powder inhaler according to claim 11, wherein each of the medicament carriers (201) comprises an elongate blister strip having a base layer (203) defining spaced-apart blister openings (205) containing medicament doses, and a cover layer (207) adhesively bonded to the base layer (203) to close the blisters, and wherein the cover layer (207) is arranged to be peeled from the base layer (203), optionally wherein the dispensing mechanism comprises:
an indexing mechanism for moving a blister of each of the elongate blister strips (201) containing the next medicament to the dispensing position; and
a peeling mechanism for peeling the cover layer from each of the blister strips (201) for placing the medicament doses in fluid communication with the air flow path ready for simultaneous inhalation by the user.

13. The dry powder inhaler according to claim 11 or 12, further comprising the medicament carriers (201), optionally wherein the dry powder inhaler comprises exactly two medicament carriers (201a, 201b), and further optionally wherein the medicament carriers (201a, 201b) are arranged in at least one replaceable cartridge received by the housing.

14. The dry powder inhaler of claim 13, wherein the medicament carriers (201a, 201b) comprise respective different medicament for simultaneous inhalation, optionally wherein the different medicaments for simultaneous inhalation comprise:
LABA or SABA in a first medicament carrier and an ICS in a second medicament carrier; or
LABA or SABA in a first medicament carrier and LAMA or SAMA in a second medicament carrier; or
LABA or SABA and LAMA or SAMA in a first medicament carrier and ICS in a second medicament carrier, or
budesonide in a first medicament carrier and formoterol in a second medicament carrier; or
beclomethasone in a first medicament carrier and formoterol in a second medicament carrier; or
fluticasone in a first medicament carrier and salmeterol in a second medicament carrier, or
fluticasone in a first medicament carrier and albuterol in a second medicament carrier, or
fluticasone in a first medicament carrier and vilanterol in a second medicament carrier, or
umeclidinium in a first medicament carrier and vilanterol in a second medicament carrier, or
two selected from umeclidinium, fluticasone and vilanterol in a first medicament carrier, the remaining medicament from umeclidinium, fluticasone and vilanterol in a second medicament carrier

15. The dry powder inhaler according to claim 14, wherein the different medicaments for simultaneous inhalation comprise:
fluticasone furoate in a first medicament carrier and vilanterol trifenatate in a second medicament carrier, or
umeclidinium bromide in a first medicament carrier and vilanterol trifenatate in a second medicament carrier, or
two selected from umeclidinium bromide, fluticasone furoate and vilanterol trifenatate in a first medicament carrier and the remaining medicament from umeclidinium bromide, fluticasone furoate and vilanterol trifenatate in a second medicament carrier.
